# EUROPEAN PATENT SPECIFICATION

(11) **EP 3 942 943 B1**
(45) Date of publication and mention of the grant of the patent: **24.04.2024**
(21) Application number: 20930645.5
(22) Date of filing: 07.05.2020
(51) Int. Cl.: A23L 33/125, A23L 33/18, A61K 8/73, A61K 8/65, A61Q 19/00

(54) **USE OF A COMPOSITION COMPRISING GALACTO-OLIGOSACCHARIDE AND COLLAGEN TRIPEPTIDE FOR IMPROVING SKIN CONDITION**
VERWENDUNG EINER ZUSAMMENSETZUNG, DIE GALACTO-OLIGOSACCHARID UND KOLLAGENTRIPEPTID UMFASST, ZUR VERBESSERUNG DES HAUTZUSTANDS
UTILISATION D'UNE COMPOSITION COMPRENANT DU GALACTO-OLIGOSACCHARIDE ET DU COLLAGÈNE TRIPEPTIDE POUR AMÉLIORER L'ÉTAT DE LA PEAU

(30) Priority: 29.04.2020 KR 20200052861
(43) Date of publication of application: 26.01.2022
(73) Proprietor: Neo Cremar Co., Ltd., Songpa-gu Seoul 05702 (KR)
(72) Inventor: KIM, Jae-Hwan, Seoul 05702 (KR); SUH, Hyung-Joo, Seoul 02841 (KR); HONG, Ki-Bae, Seoul 02841 (KR); SHIN, Jung-Cheul, Seoul 05702 (KR); KIM, Na-Ri, Seoul 05702 (KR); JO, Kyung Ae, Seoul 02841 (KR)
(74) Representative: Herrero & Asociados, S.L.
(86) International application number: PCT/KR2020/006054
(87) International publication number: WO 2021/221221

(56) References cited:
- CN-A- 108 967 800
- CN-A- 110 897 060
- KR-A- 20060 030 042
- US-A1- 2011 009 359
- SUH MIN GEUN ET AL: "Photoprotective Effect of Dietary Galacto-Oligosaccharide (GOS) in Hairless Mice via Regulation of the MAPK Signaling Pathway", MOLECULES, vol. 25, no. 1679, 6 April 2020 (2020-04-06), XP055862523, DOI: 10.3390/molecules25071679 Retrieved from the Internet: URL:https://www.ncbi.nlm.nih.gov/pmc/artic les/PMC7180589/pdf/molecules-25-01679.pdf>
- TANABE: "Oral administration of a galactooligosaccharide preparation inhibits development of atopic dermatitis-like skin lesions in NC/Nga mice", INTERNATIONAL JOURNAL OF MOLECULAR MEDICINE, vol. 25, no. 3, 22 January 2010 (2010-01-22), XP055862485, GR ISSN: 1107-3756, DOI: 10.3892/ijmm_00000349
- PYUN HEE-BONG ET AL: "Effects of Collagen Tripeptide Supplement on Photoaging and Epidermal Skin Barrier in UVB-exposed Hairless Mice", JOURNAL OF FOOD SCIENCE AND NUTRITION, KOREAN INTELLECTUAL PROPERTY OFFICE, vol. 17, no. 4, 31 December 2012 (2012-12-31), pages 245-253, XP053028621, ISSN: 2287-1098, DOI: 10.3746/PNF.2012.17.4.245
- Tanabe Soichi, Satomi Hochi: "Oral administration of a galactooligosaccharide preparation inhibits development of atopic dermatitis-like skin lesions in NC/Nga mice", INTERNATIONAL JOURNAL OF MOLECULAR MEDICINE, Spandidos Publications, GR, vol. 25, no. 3, 22 January 2010 (2010-01-22), XP055862485, GR ISSN: 1107-3756, DOI: 10.3892/ijmm_00000349
- Pyun, Hee-Bong;Kim, Minji;Park, Jieun;Sakai, Yasuo;Numata, Noriaki;Shin, Jin-Yeong;Shin, Hyun-Jung;Kim, Do-Un;Hwang, Jae-Kwan;: "Effects of Collagen Tripeptide Supplement on Photoaging and Epidermal Skin Barrier in UVB-exposed Hairless Mice", Journal of Food Science and Nutrition, Korean Intellectual Property Office, vol. 17, no. 4, 31 December 2012 (2012-12-31), pages 245-253, XP053028621, ISSN: 2287-1098, DOI: 10.3746/pnf.2012.17.4.245
- Suh Min Geun, Bae Gi Yeon, Jo Kyungae, Kim Jin Man, Hong Ki-Bae, Suh Hyung Joo: "Photoprotective Effect of Dietary Galacto-Oligosaccharide (GOS) in Hairless Mice via Regulation of the MAPK Signaling Pathway", Molecules, vol. 25, no. 1679, 6 April 2020 (2020-04-06), XP055862523, DOI: 10.3390/molecules25071679

## Description

### Technical Field

The present disclosure was made with the support of the Ministry of Small and Medium-Sized Enterprises (SMEs) and Startups of the Republic of Korea under Project No. S2611313, which was executed in the research project named "Cosmetics and Inner Beauty Materialization Using High-Purity Galacto-Oligosaccharides" in the research program titled "Industry-Academy Research Cooperation Technology Development Business" by the NEO CREMAR Co., Ltd., under the management of the Korea Technology and Information Promotion Agency for SMEs, from 01 June 2018 to 31 May 2020.

This application claims priority and the benefit of Korean Patent Application No. 10-2020-0052861 filed in the Korean Intellectual Property Office on 29 April 2020.

The present disclosure relates to food compositions and cosmetic compositions each comprising galacto-oligosaccharides (GOS) or galacto-oligosaccharides and collagen tripeptide (CTP) for improving immune functions and skin codition.

### Background Art

Skin aging is caused by a reduction in restoration ability of cells constituting the skin. The causes of skin aging are classified into intrinsic aging occurring with the progression of age and extrinsic aging (e.g., photo-aging) caused by continuous ultraviolet exposure. Intrinsic aging is clinically characterized by, with the decrease of the number of cells, a reduced amount of extracellular matrix protein fibers synthesized, loosen structures, fine wrinkles in which the structure of the stratum corneum is changed due to the loss of moisture in skin cells, reduced subcutaneous fat layers, and reduced skin moisturization. As for extrinsic aging, reactive oxygen species (ROS) generated by ultraviolet stimuli promote the production of inflammatory cytokines, form thick and deep wrinkles, cause dry skin, and reduce elasticity. When not properly removed, the reactive oxygen species, a common factor of intrinsic or exogenous aging, hinders the restoration ability of the skin to accelerate skin aging.

The patent application CN11897060A refers to a collagen drink comprising collagen tripeptide and galacto oligosaccharides for both cosmetic and health effects.

The document Suh Min Geun et al: "Photoprotective Effect of Dietary Galacto-Oligosaccharide (GOS) in Hairless Mice via Regulation of the MAPK Signaling Pathway", Molecules, vol. 25 refers to a comparative study for food supplements. GOS is tested for photoprotective effect (parameters: water holding capacity, transepidermal water loss, wrinkling). The results of GOS are compared to collagen tripeptide, which is used as positive control.

The document Tanabe: "Oral administration of a galactooligosaccharide preparation inhibits development of atopic dermatitis-like skin lesions in NC/Nga mice", International Journal of Molecular Medicine, vol. 25, n13, 22 January 2010 discloses inhibition of development of skin lesions by oral administration of GOS. The levels of pro-inflammmatory cytokines are lowered whereas the production of immunomodulatory IL-1 O is enhanced.The patent application US2011/009359 describes methods of decreasing or maintaining the ratio of Firmicutes/Bacteroidetes in a subject by administration of a composition comprising GOS.

The document Pyun Hee Bong et al: "Effects of Collagen Tripeptide Supplement on Photoaging and Epidermal Skin Barrier in UVB-exposed Hairless Mice" Journal of food science and nutrition, Korean Intellectual Property Office, vol. 17, n14, 31 December 2021, pages 245-253, describes collagen tripeptide as a functional food material. Effects on improving dry skin and anti-photoaging are mentioned.

### Summary of the invention

The invention is set out in the appended claim.

### Technical Problem

The present inventors have conducted intensive research to identify various effects of galacto-oligosaccharides (GOS) and a mixture of galacto-oligosaccharides (GOS) and collagen tripeptide (CTP) on the skin and the mechanisms thereof. As a result, the prevent inventors established the immune function improving and anti-inflammatory or anti-oxidant effects of the galacto-oligosaccharides and the immune function improving, anti-inflammatory, anti-oxidant, wrinkle alleviating, elasticity improving, skin moisturizing, and photo-aging inhibitory effects of the mixture of galacto-oligosaccharides and collagen tripeptide, and thus completed the present disclosure.

Described herein is a food composition comprising galacto-oligosaccharides (GOS) as an active ingredient for alleviating inflammation or improving immune functions.

Further described herein a food composition comprising galacto-oligosaccharides (GOS) and collagen tripeptide (CTP) as active ingredients.

Also described herein is a cosmetic composition comprising galacto-oligosaccharides or galacto-oligosaccharides and collagen tripeptide as active ingredients for improving skin condition.

### Solution to Problem

In accordance with an aspect of the present disclosure, there is provided a food composition comprising galacto-oligosaccharides (GOS) as an active ingredient for alleviating inflammation or improving immune functions.

As used herein, the term "galacto-oligosaccharides (GOS)" is a mixture of several kinds of saccharides including galacto-oligosaccharides. The galacto-oligosaccharides may include: monosaccharides of glucose and galactose; disaccharides, such as galactobiose, lactose, and allolactose; and trisaccharides including galactosyllactose and other tetra- or higher-saccharides of galacto-oligosaccharides. The galactobiose may include 4-beta-galactobiose and 6-beta-galactobiose. The allolactose may include 6-galactosyl-glucose. The beta galactosyllactose may include 3'-galactosyllactose, 4'-galactosyllactose, 6'-galactosyllactose, 4-beta-di-galactosyllactose, 4-beta-tri-galactosyllactose, 4-beta-tetra-galactosyllactose, and the like.

Specifically, the galacto-oligosaccharides used in the present disclosure include: galactobiose and lactose, which are disaccharides; and galacto-oligosaccharides, such as tri- or higher-saccharides including galactosyllactose.

The galacto-oligosaccharides may have 17-25% of total disaccharides, 26-36% of total trisaccharides, and 16-30% of total tetrasaccharides, on an anhydrous basis, and the content of galacto-oligosaccharides excluding the monosaccharides glucose and galactose and the disaccharide lactose may be 70-80% on an anhydrous basis.

The galacto-oligosaccharides are non-digestible prebiotics, and are known to contribute to immune health by promoting digestion and helping the growth of beneficial intestinal bacteria.

As used herein, the term "collagen tripeptide (CTP)" is a minimum unit of collagen composed of three amino acids. CTP is different from conventional collagens and thus is easily absorbed into the intestinal tract. The collagen tripeptide is a material with biological effects, such as healing wounds and fractures and strengthening ligaments, and used as a food material.

As used herein, the term "anti-inflammatory" or "inflammation alleviating (relieving)" effect refers to inhibiting inflammation, and the inflammation, which is one of the body's defense responses to stimuli, refers to a complex lesion developing three types: tissue degeneration, circulatory disturbance and exudation, and tissue proliferation. More specifically, inflammation is a part of innate immunity and the inflammatory response is a non-specific defense action that makes a hostile environment for microorganisms that may invade damaged tissues or wounds. In the inflammatory response, the leukocytes responsible for the immune response in the initial stage cluster to express cytokines. Therefore, the expression levels of intracellular cytokines are indexes of inflammatory response activation. Examples of the cytokines involved in inflammatory response activation include IL-6, IL-12, TNF-alpha, and the like. The cytokines are also immune markers related to the inflammatory response.

Interleukin-6 (IL-6) is a cytokine that promotes inflammatory responses and autoimmune responses in many diseases, such as diabetes, arteriosclerosis, sLE, multiple myeloma, prostate cancer, Behcet's disease, and rheumatoid arthritis. IL-12 is an interleukin that is naturally produced by cells, such as dendritic cells, macrophages, and neutrophils, in response to antigenic stimulation, and is a cytokine that acts in a wide range of infectious diseases. The tumor necrosis factor-alpha (TNF-alpha) is a cytokine that acts in a systemic inflammatory response, and plays a central role in activating inflammatory responses including acute inflammatory responses.

It is known that repeated stimulation by ultraviolet radiation causes reactive oxygen species (ROS) and promotes the production of pro-inflammatory cytokines to activate various signaling systems, thereby increasing the inflammatory response.

In an embodiment of the present disclosure, the galacto-oligosaccharides of the present disclosure have excellent effects of inhibiting the secretion of inflammatory cytokines, such as IL-6, IL-12, and TNF-alpha and thus can be advantageously used as a food composition for improving immune functions and alleviating inflammation, and prevents excessive inflammatory responses or the delay of recovery caused by excessive inflammatory responses and promotes the recovery from injury or diseases. The improvement of immune responses and the alleviation of inflammation mean, specifically, improving skin immune functions and alleviating skin inflammation, but are not limited thereto.

Examples of the skin disease associated with inflammation include atopic dermatitis, contact dermatitis, seborrheic dermatitis, psoriasis, erythematous diseases caused by radiation, chemicals, burns, or the like, acid burn, ultraviolet burn, bullous dermatosis, lichen, allergic itching, rose acne, pemphigus vulgaris, erythema multiforme exudative, erythema nodosum, balanitis, vulvovaginitis, inflammatory hair loss such as alopecia areata, cutaneous T-cell lymphoma, and the like, but are not limited thereto. The symptoms of the skin diseases associated with inflammation include erythema, papules, blisters, excessive keratinization, itching, and the like, but are not limited thereto.

The diseases associated with inflammation are not limited to skin diseases, and examples thereof include stomatitis, vasculitis, endocarditis, osteoarthritis, rheumatoid arthritis, stomatitis, or an inflammatory bowel disease. Specifically, the inflammatory bowel disease is selected from the group consisting of ulcerative colitis, Crohn's disease, intestinal Behcet's disease, indeterminate colitis, bacterial enteritis, viral enteritis, amoebic enteritis, hemorrhagic rectal ulcer, ischemic colitis, and tuberculous enteritis, but is not limited thereto. More specifically, the inflammatory bowel disease is ulcerative colitis or Crohn's disease.

Furthermore, the present disclosure provides a food composition comprising galacto-oligosaccharides (GOS) and collagen tripeptide (CTP) as active ingredients for improving skin condition.

The improving of skin condition means moisturizing the skin, soothing the skin, alleviating skin irritation, relieving skin wrinkles, inhibiting skin damage caused by ultraviolet radiation, inhibiting photo-aging, improving skin elasticity, or alleviating skin inflammation.

Therefore, according to an aspect of the present disclosure, there is provided a food composition comprising galacto-oligosaccharides (GOS) and collagen tripeptide (CTP) as active ingredients for moisturizing the skin.

The moisturization of the skin is maintained by the moisture present in the stratum corneum of the epidermis, which is the outermost layer of the skin, and the collagen present in the dermis. The moisture content of the stratum corneum is determined by the acid mantle, which is a lipid mixture produced and secreted from the epidermis, and by the natural moisturizing factor (NMF), which is a water-soluble component present in the stratum corneum. The stratum corneum of the healthy epidermis contains 15-20% of moisture, and when the moisture falls below 10%, the skin becomes dry and loses its shine and elasticity, resulting in increased wrinkles.

In the present disclosure, collagen is also called collagenous fibers, and is a main component of the extracellular matrix that accounts for 85-90% of the dermis. Collagen is a protein that is produced in the form of procollagen by the action of fibroblasts. The newly synthesized procollagen is secreted into the extracellular space of skin cells through an enzymatic reaction, to thereby form microfibrils with a triple- helix structure, and the microfibrils bind to leucine-rich small proteoglycans to form fibrils. This process is called fibrillogenesis. Consequently, the fibrils thus formed gather to form collagen fibers to provide the binding capacity and elasticity of the skin. Collagen type I accounts for most of the skin collagen. The main functions of collagen are to offer mechanical solidity to skin, confer resistance of connective tissues and the binding capacity of tissues, support cell adhesion, induce cell division and differentiation, and the like.

In an embodiment of the present disclosure, the mixture of galacto-oligosaccharides and collagen tripeptide of the present disclosure has an effect of improving the content of collagen in the dermis when consumed. As shown in a specific embodiment of the present disclosure, as a result of investigating the change in water-holding capacity before and after ultraviolet irradiation, all of the groups treated with GOS/CTP at weight ratios of 3:1 to 1:3 showed an improvement in collagen content compared with the NOR group, and showed a synergistic effect in terms of collagen content compared with the GOS and CTP alone treatment groups.

In another embodiment of the present disclosure, the mixture of galacto-oligosaccharides and collagen tripeptide of the present disclosure has effects of maintaining high moisture-holding capacity and reducing transepidermal water loss when ingested. The transepidermal water loss (TEWL) value refers to the amount of moisture evaporating from the skin, and the higher the value, the lower the moisturizing function of the skin, indicating that the barrier function characteristic to the skin has been impaired.

In an embodiment of the present disclosure, the galacto-oligosaccharides and the collagen tripeptide are contained at a weight ratio of 1-10:1-10. More specifically, the weight ratio may be 1-10:1-10, 1:1-10, 1-10:1, 1-5:1-5, 1:1-5, 1-5:1, 1-3:1-3, 1:1-3, 1-3:1, 1:10, 1:5, 1:3, 1:2, 1:1, 10:1, 5:1, 3:1, or 2:1, but is not limited thereto. The weight ratio of galacto-oligosaccharides and collagen tripeptide contained as active ingredients in the composition of the present disclosure is the same regardless of the use of the functional food composition of the present disclosure.

As shown in a specific embodiment of the present disclosure, as a result of investigating the change in water-holding capacity before and after ultraviolet irradiation, the GOS/CTP 1:1 group showed a significant increase in water-holding capacity; all the sample administration groups showed a significant decrease in transepidermal water loss; and all the GOS/CTP 3:1 to 1:3 treatment groups showed a synergistic effect in the decrease in transepidermal water loss compared with the GOS and CTP alone treatment groups.

Hyaluronan, which is present in the skin, is formed in fibroblasts and keratinocytes and can contain a considerable amount of moisture due to the presence of a lot of polysaccharides as components of mucopolysaccharides. The cycle of hyaluronan is 2-4.5 days, and combines with water having a weight 1000 times its own to control the skin barrier function and hydrate the extracellular matrix, thereby maintaining the homeostasis of water in the tissue. Hyaluronan is present in not only the dermis but also the cell gap of the epidermis, especially the stratum spinosum, but not in the stratum corneum and stratum granulosum.

Hyaluronidase (HYAL) is a hydrolytic enzyme of hyaluronan, a skin moisturizing factor.

In a specific embodiment of the present disclosure, as a result of measuring the gene expression level of the hyaluronidase HYAL, the HYAL expression level significantly increased in the control group exposed to ultraviolet radiation and tended to decrease in all the composition administration groups. Especially, as for the HAYAL expression level, all the group treated with the mixture compositions comprising GOS/CTP at ratios of 3:1, 1:1, and 1:3 showed a synergistic effect in terms of the reduction of hyaluronidase compared with the GOS and CTP alone groups.

Therefore, the mixture of galacto-oligosaccharides and collagen tripeptide of the present disclosure has an excellent skin moisturizing effect, and thus can be advantageously used as a food composition for moisturizing the skin.

According to another aspect of the present disclosure, there is provided a food composition comprising galacto-oligosaccharides (GOS) and collagen tripeptide (CTP) as active ingredients for soothing the skin or alleviating skin irritation.

In the present disclosure, the soothing of the skin refers to imparting a feeling of cooling to the skin and means an effect of soothing erythema/redness of the skin.

In the present disclosure, the alleviating of skin irritation refers to reducing the response of the skin to external and internal stimulations applied to the skin, for example, an effect of reducing the (inflammation or erythema/redness) to the stimulation applied to the skin by ultraviolet radiation.

In a specific embodiment of the present disclosure, the administration of the galacto-oligosaccharides and collagen tripeptide of the present disclosure showed an effect of significantly reducing the expression of IL-6, IL-12, and TNF-alpha, which are cytokines as immune markers related to inflammation, to the ultraviolet stimulation. In addition, as a result of evaluating the erythema index, especially, the GOS/CTP 1:3 treatment group showed an excellent erythema reduction effect.

Therefore, the galacto-oligosaccharides and collagen tripeptide of the present disclosure can be advantageously used as raw materials of a food composition for soothing the skin or alleviating skin irradiation.

Also described here is a food composition comprising galacto-oligosaccharides (GOS) and collagen tripeptide (CTP) as active ingredients for relieving skin wrinkles.

The exposure of the skin to ultraviolet radiation induces changes in physical and biochemical properties within the skin tissue, thereby causing epithelial cell proliferation, hyperpigmentation, DNA damage through the excessive generation of reactive oxygen species, abnormal activation of various enzymes and proteins, and the like. The changes within the skin tissue impair the normal skin barrier function of regulating the absorption of exogenous substances to the skin and the evaporation of water, increase the skin thickness, and damage the structure of the dermal layer, thereby ultimately causing wrinkles.

In the present disclosure, the "wrinkle relieving " effect refers to suppressing or inhibiting the generation of wrinkles in the skin or mitigating the already generated wrinkles.

In a specific embodiment of the present disclosure, the administration of the mixture of galacto-oligosaccharides and collagen tripeptide of the present disclosure showed a tendency of significant decrease in wrinkle pattern indexes (wrinkle area and maximum wrinkle depth) compared with the control group. As for the compositions, the GOS/CTP 1:3 to 1:1 mixed treatment groups synergistically decreased the maximum wrinkle depth, and all the GOS/CTP 3:1 to 1:3 mixed groups synergistically decreased the wrinkle area. Therefore, the compositions of the present disclosure can be advantageously used as food compositions for relieving skin wrinkles.

According to another aspect of the present disclosure, there is provided a food composition comprising galacto-oligosaccharides (GOS) and collagen tripeptide (CTP) as active ingredients for inhibiting skin damage caused by ultraviolet radiation.

It has been described above that the exposure of the skin to ultraviolet radiation causes damages in the skin tissue. At the time of the UV exposure for a long period of time, the epidermal thickness increases by 2-3 times and an increase in spinocellular cells, the polymorphism of keratinocytes, and the like are observed in the epidermal layer in the skin tissue. In photo-aging, the formation of the stratum corneum increases to protect the dermal layer of the skin and the skin becomes thicker, and thus the thickening of the skin due to ultraviolet radiation or the like means that the damage to the skin is large.

As shown in a specific embodiment of the present disclosure, the administration of the compositions comprising galacto-oligosaccharides and collagen tripeptide of the present disclosure decreased the significant epidermal thickness increase caused by ultraviolet irradiation, and especially, the groups treated with compositions comprising GOS/CTP at weight ratios of 1:1 and 3:1 showed a significant reduction in epidermal thickness, indicating a synergistic effect, compared with the GOS and CTP alone treatment groups. Therefore, the compositions of the present disclosure effectively inhibit skin damage caused by ultraviolet radiation, and especially show a synergistic effect in a weight range of 1:1 to 3:1.

According to another aspect of the present disclosure, there is provided a food composition comprising galacto-oligosaccharides (GOS) and collagen tripeptide (CTP) as active ingredients for inhibiting photo-aging.

By the stimulation of ultraviolet light of sunlight, reactive oxygen species (ROS) are generated, and the production of pro-inflammatory cytokines are promoted to activate several signaling systems. In addition, AP-1 is activated to inhibit TGF-β and TGF-α, thereby reducing the synthesis of collagen type I and collagen type III, and the activation of AP-1 and NF-_{Kβ} activates MMPs, especially, MMP-1, MMP-3, and MMP-9 to promote the disintegration of connective tissue in the dermis.

Collagen, which is a main component of the skin dermis, is a major factor in maintaining the elasticity and strength of the skin tissue and is a marker of skin protection against ultraviolet radiation. Collagen type I (COL1a1) accounts for 80-85% of total collagens.

In addition, matrix metalloproteinases (MMPs) are collagenases, and the increase in MMP activity promotes the degradation of collagen. The ultraviolet light increases the expression of several MMPs to increase the degradation of collagen, and damages the structure of the dermal layer, thereby ultimately causing skin wrinkles (aging).

MMPs are representative enzymes that degrade collagen, but tissue inhibitors of metalloproteinases (TIMPs) are inhibitors that inhibit the activity of MMP.

In an embodiment of the present disclosure, as a result of investigating the collagen content in the dermis, all the sample groups showed an increase in collagen content, and the collagen content significantly synergistically increased in the GOS alone intake group or the GOS/CTP mix intake groups for all the weight ratios of 1:3 to 3:1, compared with the CTP alone intake group, and especially, the most significant effect was shown in the GOS/CTP 1:3 mix intake group.

In another embodiment of the present disclosure, as a result of measuring the mRNA expression levels of the collagenase MMPs in the dermis, when the compositions comprising GOS and CTP of the present disclosure were administered, the expression levels of the MMP2/3/9/13 genes significantly decreased in the treatment with the GOS/CTP 1:1 and/or 3:1 mixture.

In still another embodiment of the present disclosure, as a result of measuring the expression levels of TIMP1 and TIMP2 genes, which are collagenase inhibitors in the dermis, when the compositions comprising GOS and CTP of the present disclosure were administered, the expression levels of TIMP1 and TIMP2 increased in all the weight ratios of GOS/CTP of 1:3 to 3:1.

Therefore, it was identified that the compositions for inhibiting photo-aging of the present disclosure had a synergistic effect in the inhibition of photo-aging in the range of GOS/CTP weight ratio of 1:3 to 3:1, more specifically, 1:1 to 3:1.

According to another aspect of the present disclosure, there is provided a food composition comprising galacto-oligosaccharides (GOS) and collagen tripeptide (CTP) as active ingredients for improving skin elasticity.

It is known that the decrease in elastin protein having a network structure of the dermal layer is also closely related to the reduction in skin elasticity, the reduction of elastin protein being caused by photo-aging or intrinsic aging.

In the present disclosure, elastin accounts for about 3-4% of the dermal layer and is an elastic fiber that affects the elasticity of the skin. According to the expression of elastin genes, a protein called tropoelastin is synthesized, and this tropoelastin is combined with different tropoelastins to finally make an elastin protein.

In an embodiment of the present disclosure, as a result of investigating the content of elastin fibers in the dermis, the compositions comprising galacto-oligosaccharides and collagen tripeptide of the present disclosure increased the content of elastin fibers in all the weight ratios compared with the control group, and especially, the administration of the compositions comprising GOS/CTP at weight ratios of 1:3 and 3:1 showed a synergistic effect in terms of the elastin fiber increasing effect compared with the GOS and CTP alone administration groups.

It is known that the elasticity of the skin is also related to the content of collagen in the dermis. The increase in collagen content was identified in all the sample groups, and the collagen content significantly synergistically increased in the GOS alone intake group or the GOS/CTP mix intake groups for all the weight ratios of 1:3 to 3:1 compared with the CTP alone intake group, and especially, the most significant effect was showed in the GOS/CTP 1:3 mix intake group.

Therefore, the mixture of galacto-oligosaccharides and collagen tripeptide of the present disclosure can be advantageously used as a food composition for improving skin elasticity, specifically, in the weight ratio range of 1:3 to 3:1 of GOS/CTP, and more specifically, at a weight ratio of 1:3 or 3:1 of GOS/CTP.

According to another aspect of the present disclosure, there is provided a food composition comprising galacto-oligosaccharides (GOS) and collagen tripeptide (CTP) as active ingredients for improving immune functions and alleviating inflammation.

According to still another aspect of the present disclosure, there is provided a food composition comprising galacto-oligosaccharides (GOS) and collagen tripeptide (CTP) as active ingredients for improving immune functions or intestinal microflora.

In the present disclosure, the inflammation alleviating (inflammation mitigating or anti-inflammatory) effect is the same as described with respect to the food composition comprising galacto-oligosaccharides as an active ingredient for improving immune functions and alleviating inflammation.

In the present disclosure, the term "immunity" is a self-defense system present in the body, and a procedure in which various kinds of materials or organisms invading from the outside of the body are recognized as foreign materials against the organism's own body and then eliminated and metabolized. The immunity protects the organism's own body from the damage caused by external stimulation or invasion of pathogenic microorganisms, but like an inflammatory response or the like, the immunity may also damage the organism's own tissues. The improvement of immune function is the action of regulating the change in immune function to restore the immune function to normality or decrease the width of the change, and is divided into the mitigation of hypersensitive immune response or the enhancement of immune function. The mitigation of hypersensitive immune response refers to inhibiting an undesirably increased immune response, such as an allergic reaction caused by an adverse reaction to a foreign substance or a response to a self-antigen or a modified self-antigen. The improvement of immune function is divided into the enhancement of immune function for the purpose of enhancing biological defense ability and the mitigation of excessive immune response for the purpose of modulating and improving sensitive immune function.

In an embodiment of the present disclosure, the improving of immune function is the mitigation of excessive immune response. The mitigation of excessive immune response includes the mitigation of organ transplant rejection and allergy, the mitigation of inflammation or inflammatory diseases, and the mitigation of autoimmune diseases.

In the present disclosure, the organ transplant rejection is, specifically, acute or chronic transplant rejection that occurs after transplantation of the heart, lung, heart and lung complex, liver, kidney, pancreas, skin, bowel, or cornea, and graft-versus-host disease after bone marrow transplantation, especially, T cell-mediated rejection after transplantation.

In the present disclosure, the allergy, autoimmune disease, inflammation, or inflammatory disease may be selected from group consisting of, but not limited thereto, skin allergy, nasal allergy, bronchial allergy, food allergy, septicemia, arteriosclerosis, bacteremia, systemic inflammatory response syndrome, multiple organ dysfunction syndrome, osteoporosis, periodontitis, systemic lupus erythematosus, rheumatoid arthritis, osteoarthritis, juvenile chronic arthritis, spondyloarthropathy, multiple sclerosis, systemic sclerosis, idiopathic inflammatory disorder of muscle, Sjogren's syndrome, systemic vasculitis, sarcoidosis, autoimmune hemolytic anemia, autoimmune thrombocytopenia, thyroiditis, diabetes mellitus, an immune-mediated renal disease, a demyelinating disease in the central nervous system or peripheral nervous system, idiopathic demyelinating polyneuropathy, Guillain-Barre syndrome, chronic inflammatory demyelinating polyneuropathy, hepatobiliary disease, infectious or autoimmune chronic active hepatitis, primary biliary cirrhosis, granulomatous hepatitis, sclerosing cholangitis, cytokine storm, inflammatory bowel disease (IBD), ulcerative colitis, Crohn's disease, irritable bowel syndrome, gluten-irritable bowel disease, Whipple's disease, autoimmune or immune-mediated skin disease, bullous skin disease erythema multiforme, contact dermatitis, psoriasis, allergic disease, asthma, allergic rhinitis, atopic dermatitis, pruritus, food sensitivity, acne, urticaria, pulmonary immune disease, eosinophilic pneumonia, idiopathic pulmonary fibrosis, and hypersensitivity pneumonitis.

As used herein, the term "pruritus" refers to any itchy condition, and includes an unpleasant sensation that causes a desire to scratch or rub the skin. Such pruritus may be largely divided into pruritus caused by skin diseases and pruritus caused by internal medicine diseases. The pruritus caused by skin diseases includes pruritus caused by atopic dermatitis, urticaria, psoriasis, pemphigus, dry skin, lichen simplex chronicus, prurigo nodularis, lichen, and the like. The pruritus caused by internal medicine diseases include pruritus caused by thyroid disease, pruritus caused by hormonal abnormalities such as diabetes, renal or uremic pruritus caused by chronic renal failure and hemodialysis, neoplastic pruritus caused by leukemia or lymphoma, cholestatic pruritus, pruritus caused by graft-versus-host disease, pruritus caused by anemia and metabolic disease, pruritus caused by acquired immunodeficiency syndrome, and the like.

Examples of biomarkers for identifying functionality related to the mitigation of immune response include immunoglobulin (IgE), prostaglandin, leukotriene, histamine, NO, iNOS, COX2, cytokines (TNF, IL, IFN), and the like.

The food composition comprising galacto-oligosaccharides and collagen tripeptide as active ingredients of the present disclosure has excellent effects of reducing the expression of the inflammatory cytokines IL-6, IL-12, and TNF-alpha. IL-6 is a cytokine that is known to be related to itching of the skin, indicating that the composition of the present disclosure can be used for prevention, alleviation, or treatment of pruritus. IL-12 is known to increase in various types of skin inflammatory responses, such as atopic dermatitis and allergic contact dermatitis. TNF-alpha is known as a cytokine that induces inflammation in rheumatoid arthritis as well as skin diseases, such as atopic dermatitis and psoriasis.

In a specific embodiment of the present disclosure, all the GOS and CTP mixtures at weight ratios of 1:3, 1:1, and 3:1 of GOS and CTP showed a synergistic effect in terms of the inflammatory cytokine secretion reducing effect, compared with the GOS and CTP alone administration groups. Therefore, the compositions of the present disclosure can be advantageously used as compositions for improving immune functions or alleviating inflammation (anti-inflammation).

Additionally, in an embodiment of the present disclosure, the improving of immune functions includes the improvement in intestinal immune functions. The improvement in intestinal immune functions is attained by suppressing harmful intestinal bacteria and increasing beneficial intestinal bacteria.

In another embodiment of the present disclosure, the improving of the intestinal microflora refers to decreasing the ratio of the phylum Firmicutes bacteria/the phylum Bacteroidetes bacteria in the intestine.

In another embodiment of the present disclosure, the improving of the intestinal microflora is increasing the genus Bifidobacterium strains.

As verified in an exemplary embodiment of the present disclosure, GOS or the GOS and CTP mixture of the present disclosure decreases the ratio of the phylum Firmicutes bacteria/the phylum Bacteroidetes bacteria in the intestine and increases the genus Bifidobacterium strains among lactic acid bacteria in the intestine, and therefore can be advantageously used as a composition for improving intestinal microbial flora.

In an embodiment of the present disclosure, the improving of the intestinal microflora is supported by the increase in short-chain fatty acids. Through the fermentation of dietary fibers in the intestine by intestinal microbes, short-chain fatty acids, mainly acetic acid, propionic acid, and butyric acid, are produced, and these short-chain fatty acids are known to be rapidly absorbed into the intestinal mucosa and used as a main energy source for colonic mucosal cells (Thompson DB, 2000).

As verified in an exemplary embodiment of the present disclosure, GOS or the GOS and CTP mixture of the present disclosure increases the total short-chain fatty acids and individual short-chain fatty acids (acetic acid, butyric acid, valeric acid, etc.) in the intestine, and through the increase in intestinal lactic acid bacteria and the decrease in harmful intestinal bacteria, the intestinal immune functions and intestinal microbial flora are improved.

In the present disclosure, the food composition may be used as various types of food additives or a health functional food. The food may be manufactured in the form of a powder, granules, a tablet, a capsule, a drink, or the like, and specifically, examples of the food may be drinks, meats, chocolates, foods, confectionery, pizzas, instant noodles, other noodles, gums, ice creams, alcohol drinks, vitamin complexes, and health supplement foods.

The content of the GOS and CTP mixture of the present disclosure contained in the food composition may be regulated as appropriate according to the form of a food, the desired use thereof, or the like, but is not particularly limited thereto. For example, the content of GOS and CTP may be 0.001 to 30 wt% or 0.01 to 20 wt% of the total food weight, and a health drink composition may have, relative to 100 ml thereof, GOS and CTP of 0.001 to 15 g, 0.02 to 10 g, or 0.3 to 1 g, but is not limited thereto.

The composition comprising the GOS and CTP mixture of the present disclosure, when prepared as a food composition, may contain ingredients that are usually added in the manufacture of foods, in addition to the GOS and CTP mixture as an active ingredient. Examples of the added ingredients include proteins, carbohydrates, fats, nutrients, seasonings, and flavoring agents. Examples of the carbohydrates are: common saccharides, such as monosaccharides (e.g., glucose and fructose), disaccharides (e.g., maltose, sucrose, and oligosaccharides), and polysaccharides (e.g., dextrin and cyclodextrin); and sugar alcohols, such as xylitol, sorbitol, and erythritol. Examples of the flavoring agents may include natural flavoring agents (thaumatin, and stevia extracts (e.g., rebaudioside A, glycyrrhizin, etc.)) and synthetic flavoring agents (saccharin, aspartame, etc.). For example, the food composition of the present disclosure, when manufactured as a drink, may contain citric acid, high-fructose corn syrup, sugar, glucose, acetic acid, malic acid, fruit juice, an Eucommia ulmoides extract, a jujube extract, a licorice extract, and the like, in addition to the extract of the present disclosure.

According to another aspect of the present disclosure, there is provided a cosmetic composition comprising i) galacto-oligosaccharides (GOS) or ii) galacto-oligosaccharides and collagen tripeptide (CTP) for improving skin condition.

As verified in the above-described exemplary embodiments, the galacto-oligosaccharides (GOS) i) or the galacto-oligosaccharides and collagen tripeptide (CTP) ii) as active ingredients of the present disclosure significantly decreased the expression of cytokines related to inflammatory responses and decreased transepidermal water loss, erythema index, or the like. Therefore, the composition of the present disclosure has excellent effects of reinforcing skin barrier functions, protecting the skin from skin stimulation, and mitigating skin irritation.

The improving of skin condition of the present disclosure is as defined in the above-described compositions.

In an embodiment of the present disclosure, the composition of the present disclosure may contain not only the galacto-oligosaccharides (GOS) i) or galacto-oligosaccharides and collagen tripeptide (CTP) ii) as active ingredients of the present disclosure, but also the ingredients that are commonly used in the cosmetic composition, for example, carriers, and typical adjuvants, such as an antioxidant, a stabilizer, a solubilizer, a vitamin, a pigment, and a flavoring.

As the carriers, purified water, monohydric alcohols (ethanol or propyl alcohol), polyhydric alcohols (glycerol, 1,3-butylene glycol, or propylene glycol), higher fatty acids (palmitic acid or linolenic acid), oils (wheat germ oil, camellia oil, jojoba oil, olive oil, squalene, sunflower oil, macadamia peanut oil, avocado oil, soybean water-added lecithin or fatty acid glyceride), and the like may be used, but are not limited thereto. As needed, a surfactant, a sterilizer, an antioxidant, an ultraviolet light absorbent, an anti-inflammatory agent, and a refreshing agent may be added.

As the surfactant, polyoxyethylene, hydrogenated castor oil, polyoxyethylene, oleyl ether, monooleic acid polyoxyethylene, polyoxyethylene, glyceryl monostearate, monostearic acid sorbitan, monooleic acid polyoxyethylene, sorbitan, sucrose fatty acid ester, monolauric acid hexaglycerin, polyoxyethylene reduced lanolin, POE, glyceryl pyroglutamic acid, isostearic acid, diester, N-acetyl glutamine, isostearyl ester, and the like may be used.

As the sterilizer, hinokithiol, triclosan, chlorhexidine gluconic acid salt, phenoxy ethanol, resorcin, isopropylmethylphenol, azulene, salicylic acid, zincpyritaon, and the like may be used.

As the anti-oxidant, any one of butylhydroxyanisole, gallic acid, propyl gallate, and erythorbic acid may be used.

As the ultraviolet light absorbent, any one from benzophenones such as dihydroxybenzophenone, melanin, para-aminobenzoic acid ethyl, para-dimethylamino benzoic acid 2-ethylhexyl ester, cynocite, para-methoxy cinnamic acid 2-ethylhexyl ester, 2-(2-hydroxy-5-methylphenyl)benzotriazole, urocanic acid, metal oxide microparticles, and the like may be used.

As the anti-inflammatory agent, dipotassium glycyrrhizinate, allantoin, or the like may be used, and as the refreshing agent, capsicum tincture, 1-menthol, or the like may be used.

In another embodiment of the present disclosure, the cosmetic composition of the present disclosure may be prepared in a formulation selected from the group consisting of a solution, an ointment for external use, a gel, a cream, a foam, a nourishing toner, a softening toner, a pack, a softener, a milky lotion, a makeup base, an essence, an ampoule, a hair ampoule, a scalp treatment, a hair tonic, a hair conditioner, a hair treatment, a hair lotion, a hair shampoo, a hair conditioner, a conditioner shampoo, a hair nourishing lotion, a hair gel, a hair wax, a hair spray, a dye, a soap, a liquid cleanser, a bath agent, a sunscreen cream, a sun oil, a suspension, an emulsion, a paste, a gel, a lotion, a powder, a soap, a surfactant-containing cleansing, an oil, a powder foundation, an emulsion foundation, a wax foundation, a patch, and a spray, but is not limited thereto.

According to still another aspect of the present disclosure, there is provided a method for improving skin condition, the method comprising administering to a subject a composition comprising i) galacto-oligosaccharides (GOS), or ii) galacto-oligosaccharides and collagen tripeptide (CTP).

The improving of skin condition is moisturizing the skin, soothing the skin, alleviating skin irritation, relieving skin wrinkles, inhibiting skin damage caused by ultraviolet radiation, inhibiting photo-aging, improving skin elasticity, or anti-inflammation, but is not limited thereto.

As used herein, the term "administration" or "administer" refers to the direct administration of a therapeutically effective amount of the composition of the present disclosure to a subject (i.e., an individual) suffering a respiratory disease, thereby forming the same amount of the composition in the body of the subject.

As used herein, the term "subject" is a mammal including a human, a mouse, a rat, a guinea pig, a dog, a cat, a horse, a cow, a pig, a monkey, a chimpanzee, a baboon, a rhesus monkey, and the like. Most specifically, the subject of the present disclosure is a human.

The method for improving skin condition of the present disclosure includes administering a food composition having various uses according to an aspect of the present disclosure, and thus a description of overlapping contents therebetween is omitted to avoid excessive redundancy of the present specification.

### Advantageous Effects of the Disclosure

The present disclosure provides functional food compositions comprising galacto-oligosaccharides (GOS) or galacto-oligosaccharides (GOS) and collagen tripeptide (CTP) as active ingredients. The compositions of the present disclosure have excellent effects associated with the uses of photo-aging inhibition, skin wrinkle relief, skin elasticity improvement, anti-oxidation, anti-inflammation, or skin moisturization, and thus can be advantageously used as functional food compositions for the above-mentioned uses.

### Brief Description of Drawings

FIG. 1A shows food intake for each experimental group of the present disclosure.
FIG. 1B shows water intake for each experimental group of the present disclosure.
FIG. 2A shows skin water-holding capacity for each experimental group of the present disclosure.
FIG. 2B shows a change in skin water-holding capacity for each experimental group of the present disclosure.
FIG. 3A shows transepidermal water loss for each experimental group of the present disclosure.
FIG. 3B shows a change in transepidermal water loss for each experimental group of the present disclosure.
FIG. 4A shows the erythema value for each experimental group of the present disclosure.
FIG. 4B shows a change in erythema value for each experimental group of the present disclosure.
FIG. 5 shows the visual and photographing observations of mouse dorsal skin for each experimental group of the present disclosure.
FIG. 6A shows the total skin wrinkle area for each experimental group of the present disclosure.
FIG. 6B shows the maximum wrinkle depth for each experimental group of the present disclosure.
FIG. 7 shows the results of H&E staining of the mouse skin for each experimental group of the present disclosure.
FIG. 8 shows the epidermal thickness measured from the results of H&E staining of the mouse skin for each experimental group of the present disclosure.
FIG. 9 shows the results of IHC staining of collagen in the mouse skin for each experimental group of the present disclosure.

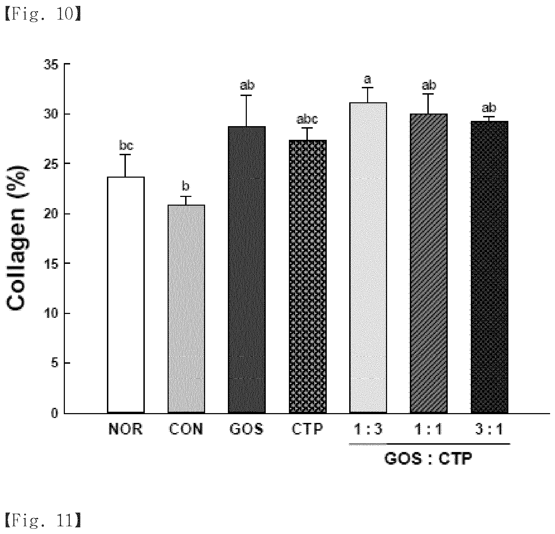

FIG. 10 shows the collagen value measured from the results of IHC staining of collagen in the mouse skin for each experimental group of the present disclosure.
FIG. 11 shows the results of measuring the amount of elastin in dermis of the mouse for each experimental group of the present disclosure.
FIGS. 12 to 15 show the mRNA expression levels of MMP2/3/9/13 in the skin tissue for each experimental group of the present disclosure.
FIGS. 16 and 17 show the mRNA expression levels of TIMP1 and TMP2 in the skin tissue for each experimental group of the present disclosure.
FIG. 18 shows the mRNA expression level of collagen type I (COL1a1) in the skin tissue for each experimental group of the present disclosure.
FIG. 19 shows the mRNA expression level of hyaluronidase in the skin tissue for each experimental group of the present disclosure.
FIGS. 20 to 22 show the levels of inflammatory cytokines secreted in the skin tissue for each experimental group of the present disclosure (FIG. 20: IL-6, FIG. 21: IL-12, and FIG. 22: TNF-alpha).
FIGS. 23 to 27 show short-chain fatty acids (SCFAs) for each experimental group of the present disclosure (FIG. 23: Acetic acid, FIG. 24: Propionic acid, FIG. 25: Butyric acid, FIG. 26: Valeic acid, and FIG. 27: Total SCFA).
FIG. 28 shows the results of UPGMA clustering analysis to analyze the populations of dermal and intestinal health functional microbiomes for each experimental group of the present disclosure.
FIGS. 29 to 31 show the distributions of Bacteroidetes and Firmicutes in the intestinal microbiomes for each experimental group of the present disclosure.

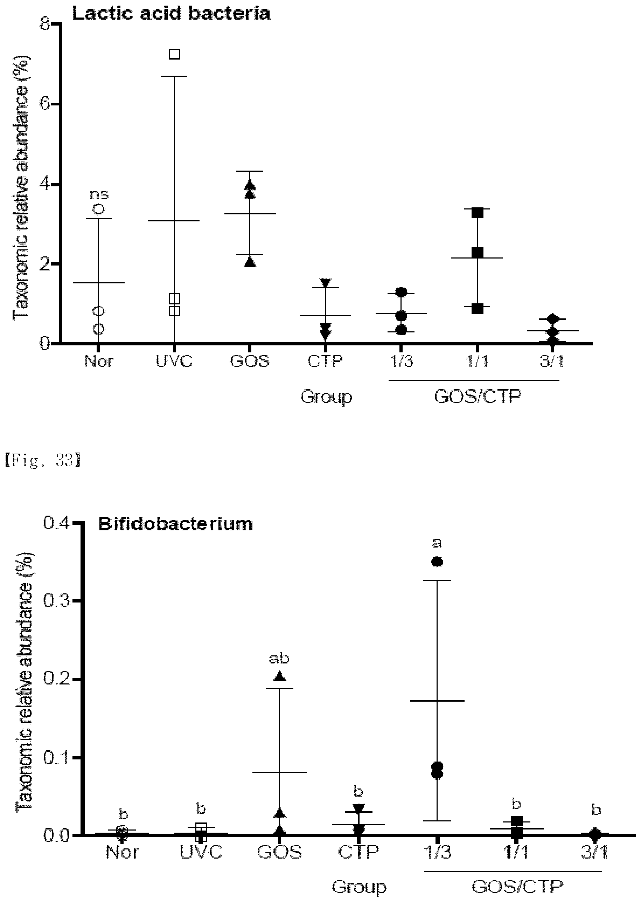

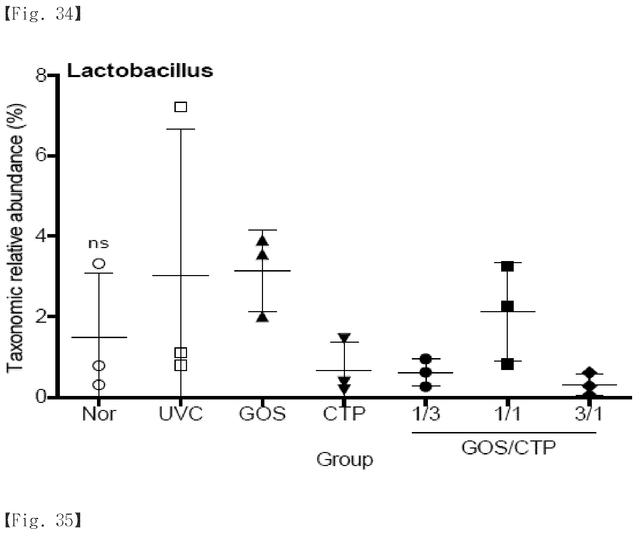

FIGS. 32 to 35 show the distributions of Bacteroidetes and Firmicutes in the intestinal microbiomes for each experimental group of the present disclosure.
FIGS. 36 to 40 show the distributions of the phyla and families of intestinal microorganisms in the intestinal microbiomes for each experimental group of the present disclosure.
FIGS. 41 to 44 show the distributions of the genera of intestinal microorganisms in the intestinal microbiomes for each experimental group of the present disclosure.

### Examples

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Materials and Reagents

The galacto-oligosaccharides (GOS) and collagen-tripeptide (CTP) used were donated by Neo Crema Co., Ltd. The galacto-oligosaccharides provided were composed of 0.5% of glucose, 1.8% of galactose, 21.7% of lactose, 20.4% of disaccharides in galacto-oligosaccharide components, 30.6% of trisaccharides in galacto-oligosaccharide components, and 25.0% of tetra- or higher-saccharides in galacto-oligosaccharide components. GOS and CTP used in the present disclosure were dissolved alone or as mixtures at different ratios in distilled water before use.

### Animals

A total of seven experimental groups (six animals per group) were defined as a normal control group (normal, not UVB-induced), a negative control group (control, UVB-irradiated), a GOS alone treatment group (GOS 200 mg/kg, UVB-irradiated) and a CTP alone treatment group (CTP 200 mg/kg, UVB-irradiated), a GOS/CTP 1:3 mixed treatment group (GOS/CTP 50:150 mg/kg, UVB-irradiated), a GOS/CTP 1:1 mixed treatment group (100:100 mg/kg, UVB-irradiated), and a GOS/CTP 3:1 mixed treatment group (GOS/CTP 150:50 mg/kg, UVB-irradiated).

The experimental animals, male SKH-1 hairless mice (SkH: HR-1), were purchased from Orient Bio, and housed. The mice were kept at a temperature of 22±2°C with a relative humidity of 50±10% under a 12-h day/night cycle. The mice were given free access to food and water.

All the experiments were approved by the Institutional Animal Care Use Committee of Korea University, and conducted according to the Guide for the Care and Use of Laboratory Animals (NRC).

The mice were acclimated for one week, and then photo-aging was induced in the mice of six groups except for the normal control group (NOR) by exposing their dorsal skin to ultraviolet light three times a week for 8 weeks through the ultraviolet irradiation device (BLX-E254, Vilber Lourmat, France) equipped with the UVB-ultraviolet lamp (T-8M; Vilber Lourmat, France) (Hong et al., 2015). As for the dosage of ultraviolet irradiation, the irradiation dosage inside the ultraviolet irradiation device was measured by the UV light meter (UV-340; Lutron, Taiwan) and adjusted, and then the UVB dosage was controlled as shown in Table 1.

**TABLE 1**

| Week | 1 | 2 | 3 | 4-8 |
|---|---|---|---|---|
| UVB dosage | 1 MED | 2 MED | 3 MED | 4 MED |

| | | | | |
|---|---|---|---|---|
| *1 MED = 75 mJ/cm² | | | | |

For 8 weeks from the start date of UV irradiation, the normal control group (NOR) and the negative control group (CON) were orally administered only drinking water, and the sample treatment groups were orally administered samples mixed with drinking water according to the concentration after being weighed. After the ending of 8 weeks of the irradiation of ultraviolet light and the administration of GOS and CPT alone or mixture samples, each mouse was sacrificed.

### Example 1: Water intake and food intake

In order to investigate the effect of the composition of the present disclosure on food intake and water intake of the mice, the present inventors measured food intake and water intake once a week while irradiating the mice with ultraviolet light and administering samples to the mice.

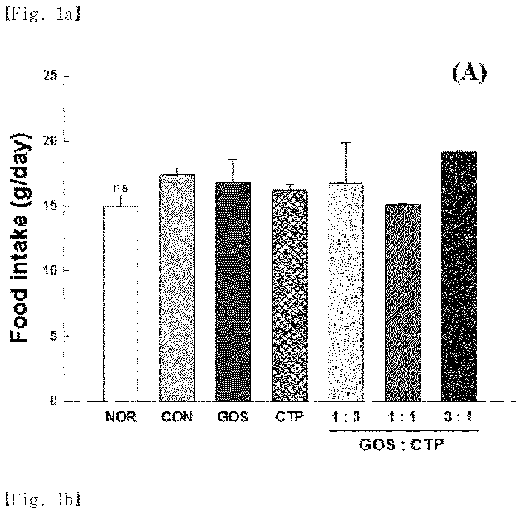

FIG. 1A shows food intake for each experiment group of the present disclosure.

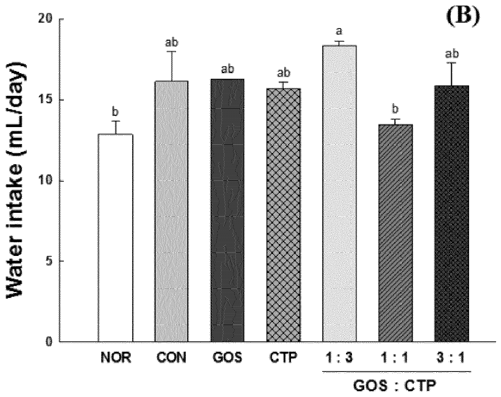

FIG. 1B shows water intake for each experiment group of the present disclosure.

As shown in FIG. 1A, the GOS/CTP 3:1 group showed a food intake of 19.14 g/day, which was a tendency of increase compared with 16.62 g/day, which is the average food intake of all the experimental groups, but there was no significant difference between the groups.

As shown in FIG. 1B, the water intake increased in the sample administration groups with UVB irradiation, except for the GOS/CTP 1:1 group, compared with the NOR group, and of these, the water intake of the GOS/CTP 1:3 group was 18.36 ml/day, indicating a significantly high value (p<0.05).

### Example 2: Evaluation of skin water-holding capacity, transepidermal water loss (TEWL), and erythema

In order to investigate the effect of the compositions of the present disclosure on skin condition, the present inventors measured skin water-holding capacity, transepidermal water loss (TEWL), and erythema of the dorsal skin of the mice, irradiated with ultraviolet light, by using Multi Probe Adapter^{®} MPA 6 (Courage und Khazaka, Germany). The skin measurement was performed on all the groups before the start of ultraviolet irradiation, and the skin measurement was performed every week from 4 weeks after ultraviolet irradiation to confirm a significant difference in skin measurement results between the NOR group and the CON group. As for the skin measurement results, the comparison between the last measurement result on the day before sacrifice and the measurement result on the initial skin before ultraviolet irradiation and the last measurement result are shown in the graphs.

### SKIN WATER-HOLDING CAPACITY

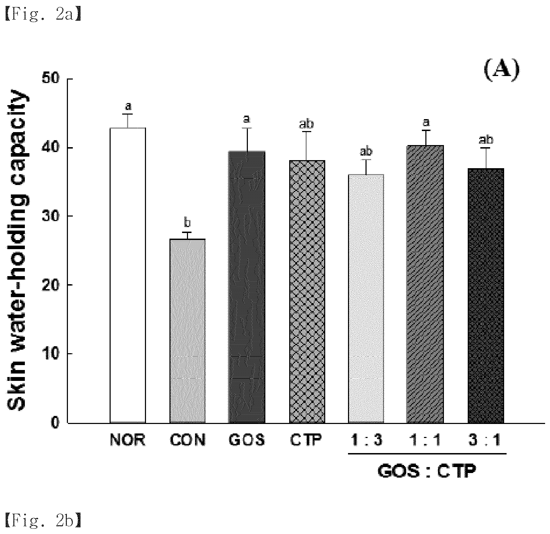

FIG. 2A shows skin water-holding capacity for each experiment group of the present disclosure.

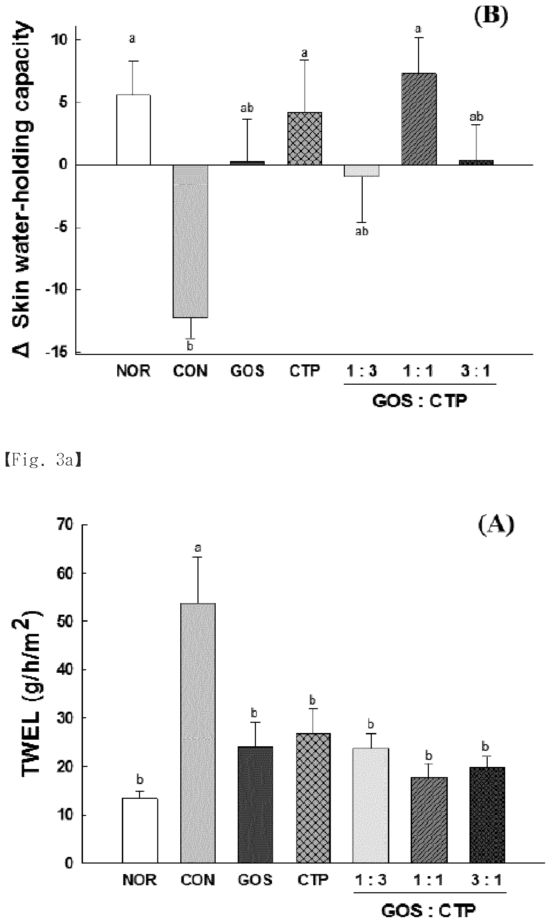

FIG. 2B shows a change in skin water-holding capacity for each experiment group of the present disclosure.

As shown in FIG. 2A, the water-holding capacity significantly decreased by about 1.61 times in the negative control group (CON) compared with the normal group (NOR), and the water-holding capacity increased in all the sample administration groups compared with the CON group. Especially, the water-holding capacity significantly increased in the GOS group and the GOS/CTP 1:1 group (p<0.05).

In addition, as shown in FIG. 2B, as a result of investigating the change in water-holding capacity before and after ultraviolet irradiation, a significant decrease was identified in the CON group compared with the NOR group. The change in water-holding capacity significantly increased in the CTP alone treatment and the GOS/CTP 1:1 group, and especially, the GOS/CTP 1:1 group showed a change in water-holding capacity at a level similar to that of NOR (p<0.05).

### Transepidermal water loss

FIG. 3A shows transepidermal water loss for each experiment group of the present disclosure.

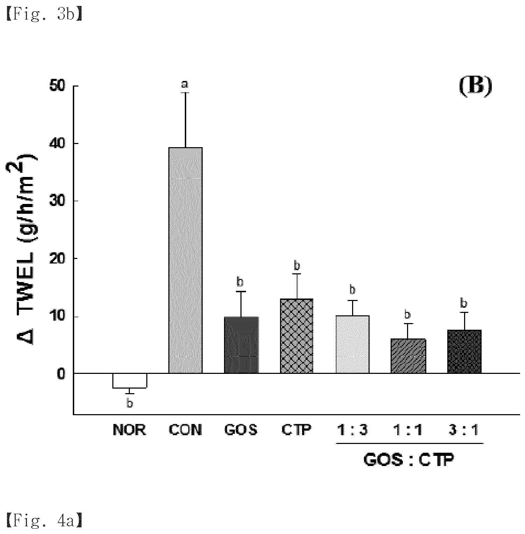

FIG. 3B shows a change in transepidermal water loss for each experiment group of the present disclosure.

As shown in FIG. 3A, the transepidermal water loss significantly increased by about 3.99 times in the negative group (CON) compared with the normal group (NOR) (p<0.05). All the sample administration groups showed a significant decrease in transepidermal water loss compared with CON, and especially, the GOS/CTP 1:1 treatment group showed a change in transepidermal water loss at a level similar to that of the NOR group (p<0.05).

In addition, as shown in FIG. 3B, as a result of investigating a change in transepidermal water loss before and after ultraviolet irradiation, the all the groups with UVB irradiation except for the NOR group showed a tendency of increase, and the all the sample administration groups showed a significant decrease compared with the CON group (p<0.05).

### Erythema value

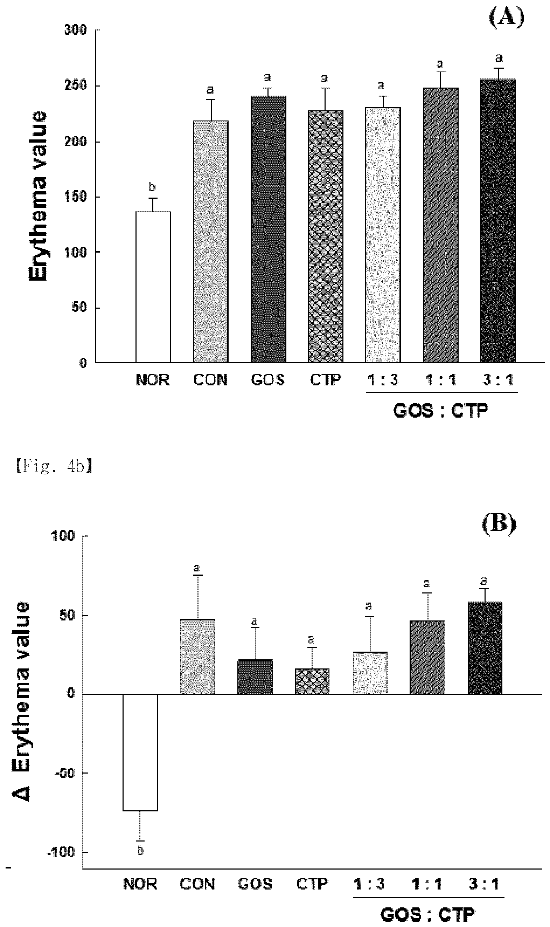

As shown in FIG. 4A, the erythema value significantly increased by about 1.60 times in the CON group compared with the NOR group (p<0.05), and the erythema values in the sample administration groups showed no significant change compared with CON.

In addition, as shown in FIG. 4B, as a result of investigating the change in erythema value before and after ultraviolet irradiation, the erythema value significantly increased in all the groups with UVB irradiation except for NOR (p<0.05). Of these, the change in erythema value tended to decrease in the GOS and CTP alone groups and the GOS/CTP 1:3 mixture group compared with the CON group, but there was no significant difference.

It was identified from the above results that all the water-holding capacity, transepidermal water loss, and erythema value showed positive activity in the intake of GOS/CTP at mixing ratios of 1:1 and 3:1 compared with the intake of COS and CTP alone. It can be therefore seen that the GOS and CTP mixed composition of the present disclosure can prevent the skin water loss and skin barrier damage, which are caused by ultraviolet light, and can help the normal functioning of the skin barrier.

### Example 3: Organ weights

In order to investigate the effect of the compositions of the present disclosure on organ weights, the present inventors harvested organs (liver, heart, kidney, spleen) to determine weights thereof to identify the toxicity and normality or abnormality after the intake of GOS and CTP samples. At the end of the experiment, the respective organs were harvested, and the weights (mg) of the harvested liver, heart, kidney, and spleen were calculated considering the body weights (g) of mice in each experimental group. The results are shown in Table 2.

**TABLE 2**

| mg/g of body weight | Liver | Heart | Kidney | Spleen |
|---|---|---|---|---|
| NOR | 5.59±0.18 | 0.50±0.03 | 2.08±0.12 | 0.27±0.02 |
| CON | 5.86±0.13 | 0.60±0.05 | 1.97±0.11 | 0.34±0.02 |
| GOS | 5.49±0.31 | 0.50±0.01 | 1.93±0.09 | 0.31±0.03 |
| CTP | 4.94±0.16 | 0.48±0.01 | 1.91±0.06 | 0.14±0.01 |
| 1:3 | 5.27±0.33 | 0.56±0.01 | 2.08±0.10 | 0.12±0.01 |
| 1:1 | 5.14±0.10 | 0.50±0.02 | 1.92±0.07 | 0.12±0.01 |
| 3:1 | 4.37±0.25 | 0.46±0.03 | 1.76±0.10 | 0.11±0.01 |

As shown in Table 2 above, the weights of the kidney and spleen showed no significant differences among the experimental groups, and the weights of the liver and heart showed some significant differences thereamong, but had no relative differences compared with the NOR group.

### Example 4: Visual observation of skin and analysis of skin replicas

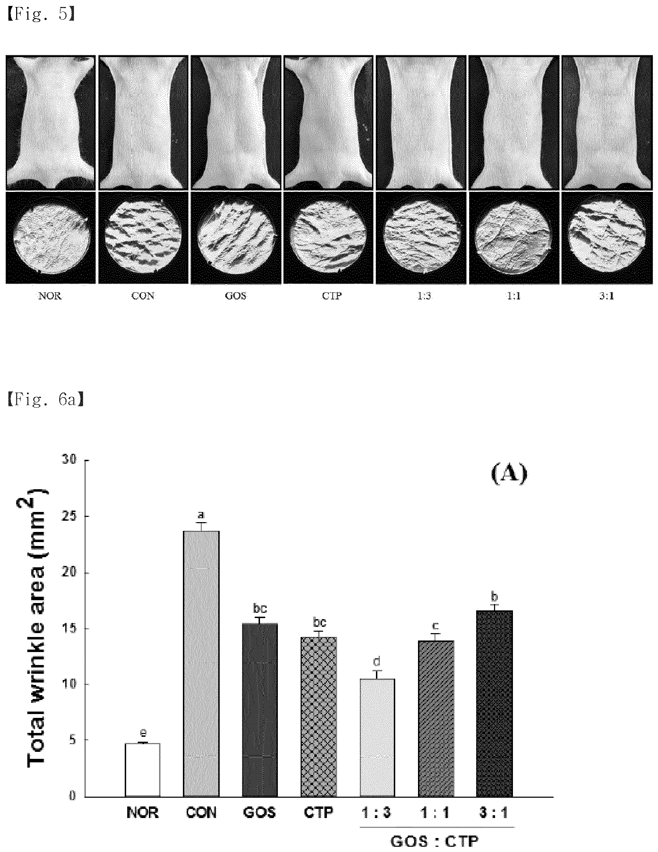

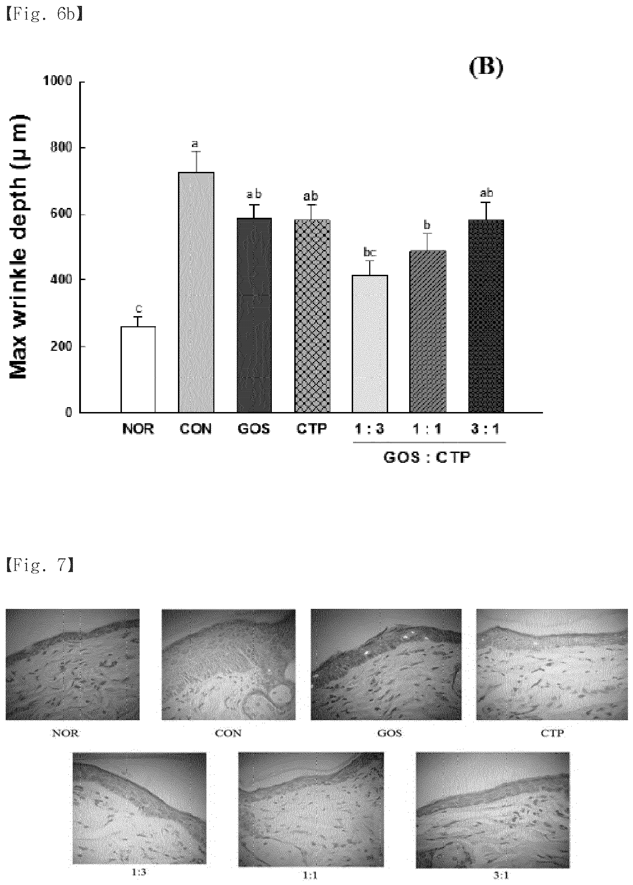

In order to investigate the wrinkle relieving effect of the compositions of the present disclosure, the present inventors photographed dorsal skin of the mice, and analyzed the area, number, length, depth, and the like of wrinkles by using replicas obtained through Visioline (VL650; CK electronic GmbH, Germany) for analysis of wrinkle patterns. The results are shown in FIGS. 5 and 6A and 6B.

As a result of analyzing the indicators of wrinkle patterns through FIGS. 6A and 6B, the negative control group (CON) showed significant increases in wrinkle area and depth compared with the normal control group (NOR) (p<0.05). The administration of the compositions of the present disclosure showed significant decrease tendencies in the indicators of wrinkle patterns compared with CON, and especially, among the compositions, the GOS/CTP 1:3 mixed treatment group showed significant decreases in wrinkle area and maximum wrinkle depth (p<0.05).

It was therefore identified that the intake of GOS and the GOS and CTP mixed composition of the present disclosure had an inhibitory effect on winkle generation due to UVB, and especially, the intake of the GOS and CTP mixed at a ratio of 1:3 showed a significant effect on wrinkle relief.

It was identified from the above results that the skin wrinkles generated by ultraviolet light decreased even in the intake of GOS and CTP alone, but the intake of GOS/CTP at a ratio of 1:3 had an effect of most significantly reducing the wrinkles generated due to photo-aging.

### Example 5: Histopathological analysis of skin - epidermal thickness and collagen measurement

In order to investigate the effects of the compositions of the present disclosure on epidermal thickness and collagen expression, the present inventors, after the ultraviolet irradiation and composition administration experiments, collected the dorsal skin of all the mice and placed the dorsal skin in 10% formalin, and then performed hematoxylin and eosin (H&E) staining whereby histopathological changes can be identified and immunohistochemistry (IHC) staining whereby the collagen present in dermis can be identified. The tissues that have been stained were photographed by a microscope, imaged, and then analyzed.

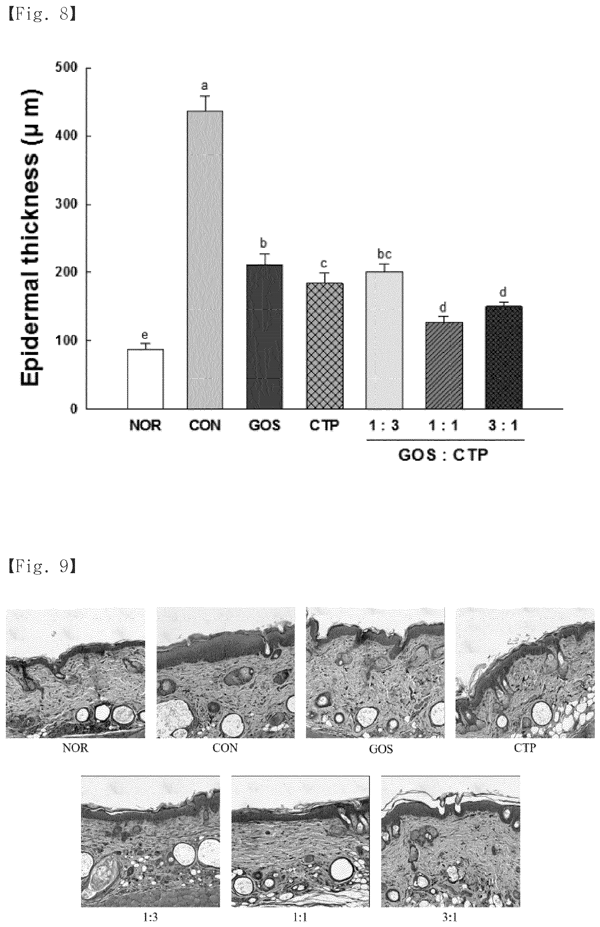

As shown in FIGS. 7 and 8, the H&E staining results identified that the epidermal thickness significantly increased in the UVB irradiation groups compared with the normal control group (NOR) (p<0.05). It was also identified that the epidermal thickness significantly decreased in all the sample administration groups compared with the CON group, and especially, the GOS/CTP 1:1 and 3:1 mixed treatment groups showed a tendency of significant decrease in epidermal thickness compared with the material alone treatment groups (p<0.05).

In the present experiment, as a result of skin histopathological observation, the epidermal thickness increased by about 4 times in the UV irradiation group compared with the control group, and the thickness tended to decrease in the mixed treatment groups compared with the GOS and CTP alone treatment groups (FIGS. 7 and 8).

Moreover, as a result of IHC staining of collagen, the proportion of collagen in dermis significantly decreased in the negative control group (CON) compared with the normal group (NOR) (p<0.05, FIG. 7). The proportion of collagen in dermis significantly increased in all the sample administration groups compared with the CON group, and especially, the proportion of collagen in dermis most significantly increased in the GOS/CTP 1:3 mixed treatment group (p<0.05).

### Example 6: Elastin measurement

Elastin, a component of the skin dermis, is an elastic substance that affects the elasticity of the skin tissue, and when the skin is photo-aged, elastin is decreased in number and diameter, resulting in the reduction in skin elasticity. In order to investigate the effect of the compositions of the present disclosure on skin elasticity, the present inventors extracted about 10 mg of the skin tissue for each experimental group, and then the amount of elastin in the skin tissue was measured by using a method provided from the Fastin Elastin assay (F2000; Biocolor, UK).

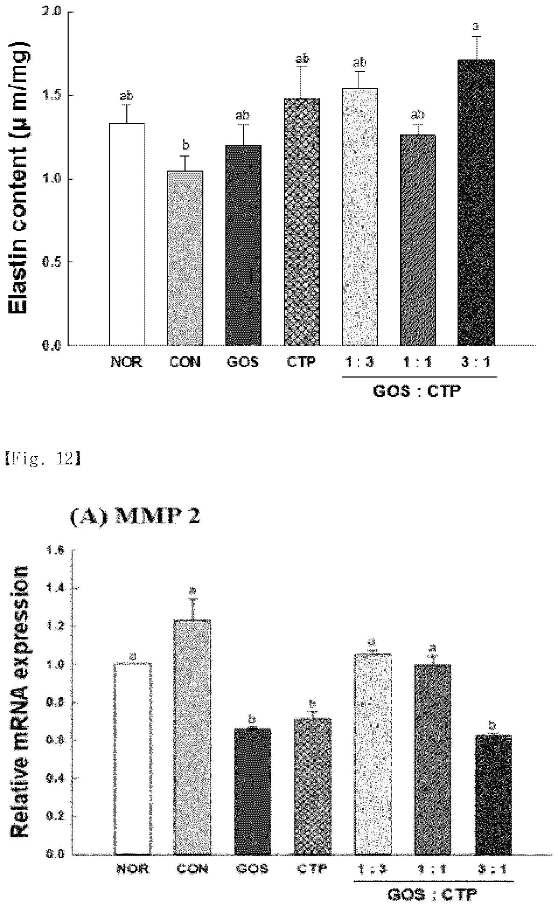

As shown in FIG. 11, the elastin content tended to increase in all the groups administered the compositions of the present disclosure, compared with the CON group, and especially, significantly increased in the treatment with the GOS/CTP 3:1 mixture compared with the CON group (p<0.05).

It was identified from the above results that the oral administration of not only the composition comprising GOS alone but also the composition comprising both GOS and CTP, especially, the composition comprising GOS/CTP at a mixing ratio of 3:1 increased the elastin content decreased by UV, and thus an elasticity enhancement effect can be expected through the improvement of the net structure of the dermal layer of the skin.

### Example 7: Analysis of expression levels of skin-related genes using qRT-PCR

In order to investigate the effects of the compositions of the present disclosure on the expression of skin condition-related genes, the present inventors measured the expression levels of collagen type I, matrix metalloproteinase (MMP) 2/3/9, TIMP1/2, hyaluronidase (HYAL), and NF-K in comparison with GAPDH.

Specifically, about 100 mg of the skin tissue was collected from each mouse of the experimental groups administered the compositions of the present disclosure, and total RNA was isolated using TRIzol reagent. To the isolated RNA was added 50 µM Oligo-(dT) primer and 10 mM dNTP mix, followed by reaction at 65°C for 5 minutes, and then 5X RT Buffer, 0.1 M DTT, RNaseOUT, SuperScript III, and 25 mM MgCl2 were added with mixing, followed by reaction under conditions of 50°C for 50 minutes and 85°C for 5 minutes. RNase H was added and incubated at 37°C for 20 minutes, thereby synthesizing cDNA from mRNA. To perform PCR, 1 µL of the synthesized cDNA was mixed with primers, SYBR green gene expression master mix, and DEPC water, followed by qPCR. The primers for each target gene are shown in FIG. 3. The expression level of each target gene was indicated as a relative expression level compared with the NOR group.

**TABLE 3**

| Target qene | Classification | PCR primer sequence (5' to 3') | SEQ ID NO |
|---|---|---|---|
| MMP2 | F | CAA GGA TGG ACT CCT GGC ACA T | 1 |
| | R | TAC TCG CCA TCA GCG TTC CCA T | 2 |
| MMP3 | F | TTC TGG GCT ATA CGA GGG CA | 3 |
| | R | CTT CTT CAC GGT TGC AGG GA | 4 |
| MMP9 | F | GCT GAC TAC GAT AAG GAC GGC A | 5 |
| | R | TAG TGG TGC AGG CAG AGT AGG A | 6 |
| TIMP1 | F | TCT TGG TTC CCT GGC GTA CTC T | 7 |
| | R | GTG AGT GTC ACT CTC CAG TTT GC | 8 |
| TIMP2 | F | AGC CAA AGC AGT GAG CGA GAA G | 9 |
| | R | GCC GTG TAG ATA AAC TCG ATG TC | 10 |
| COL1a1 | F | CCT CAG GGT ATT GCT GGA CAA C | 11 |
| | R | CAG AAG GAC CTT GTT TGC CAG G | 12 |
| HYAL | F | AAG TAC CAA GGA ATC ATG CC | 13 |
| | R | CTC AGG ATA ACT TGG ATG GC | 14 |
| NF-Kb | F | TTG TGC CAA GAG TGA TGA CG | 15 |
| | R | CTG AAA TCC CAT GTC CTG CT | 16 |
| GAPDH | F | CAT CAC TGC CAC CCA GAA GAC TG | 17 |
| | R | ATG CCA GTG AGC TTC CCG TTC AG | 18 |

### Metalloproteinases (MMPs)

As a result of measuring the gene expression level of the collagenase MMP 2, the expression level of MMP 2 increased by about 1.21 times in the CON group compared with the NOR group and tended to decrease in all the sample administration groups (FIG. 12). Especially, the expression level significantly decreased in the GOS and CTP alone groups and the GOS/CTP 3:1 mixture treatment group compared with CON (p<0.05).

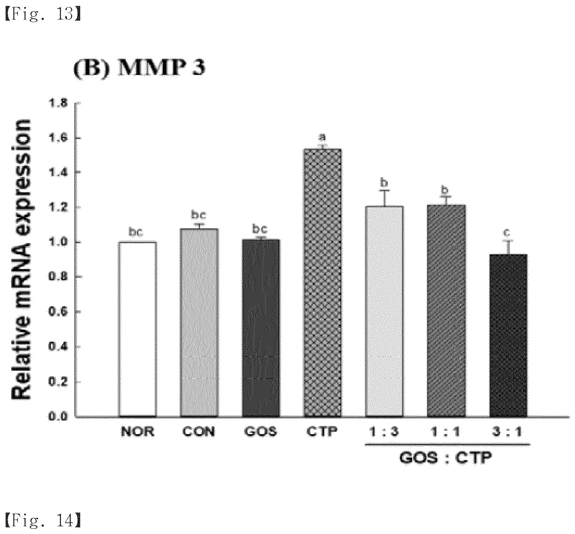

As a result of measuring the gene expression level of MMP 3, the gene expression level of MMP 3 tended to decrease in the GOS alone group and the GOS/CTP 3:1 mixture treatment group compared with the CON group, but there was no significant difference between the groups. (FIG. 13).

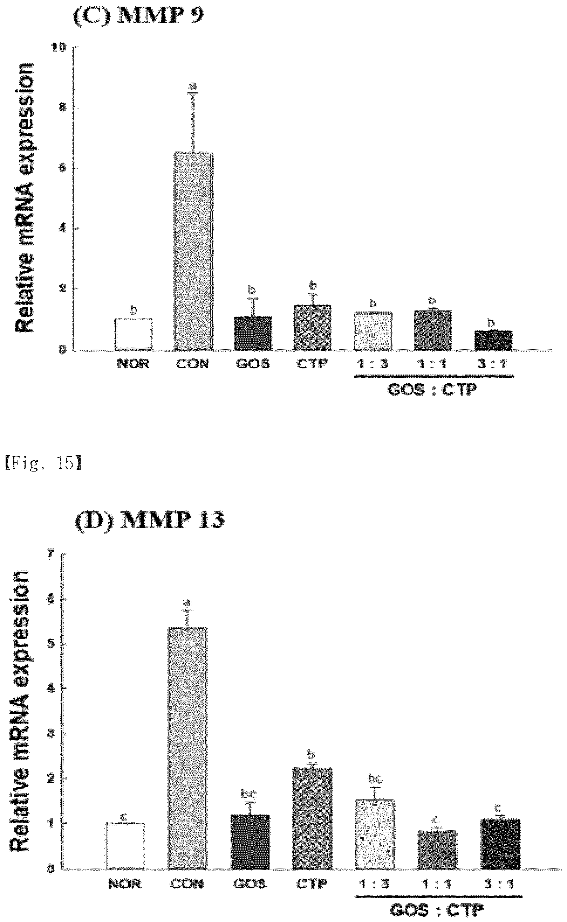

As a result of measuring the gene expression level of MMP 9 to observe the indicator of extracellular matrix degradation capacity of the skin tissue, the gene expression level of MMP 9 significantly increased by about 6.31 times in the CON group compared with the NOR group, and significantly decreased in all the sample administration groups compared with the CON group (p<0.05, FIG. 14). Among the sample administration groups, the GOS/CTP mixture treatment groups showed a decrease in expression level compared with the GOS and CTP alone treatment groups, and especially, the treatment with the 3:1 mixture showed a significant decrease compared with CON to show a similar level to that in the NOR group (p<0.05).

The gene expression level of MMP 13 significantly increased by about 5.42 times in the CON group compared with the NOR group, and significantly decreased in all the sample administration groups compared with the CON group (p<0.05, FIG. 15). Especially, the treatment with the GOS/CTP 1:1 and 3:1 mixtures showed a significant decrease compared with CON to show a similar level as that in the NOR group (p<0.05).

It was identified from the above results that the expression of MMPs induced by photo-aging decreased by the treatment with the GOS and CTP mixtures rather than the treatment with GOS and CTP alone, and especially, the expression of MMP-13, which plays an important role in wrinkle generation at the time of photo-aging, significantly decreased in the GOS/CTP i:1 treatment group.

### Tissue inhibitor of metalloproteinases (TIMPs)

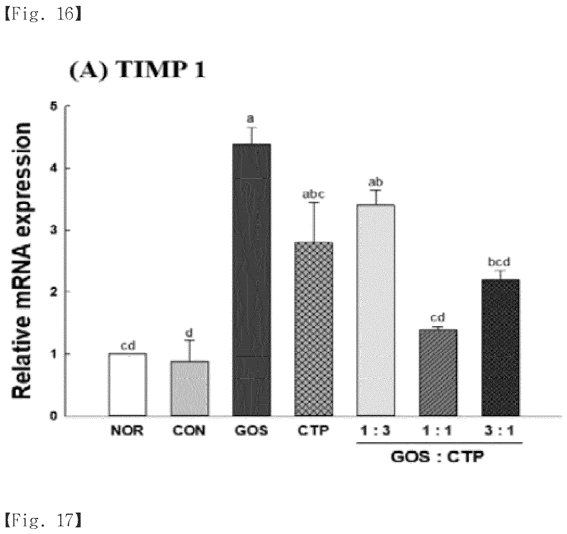

As a result of measuring the gene expression level of TIMP 1, the expression level of TIMP 1 significantly increased in all the sample administration groups compared with the CON group (p<0.05, FIG. 16). Especially, the expression level of TIMP 1 significantly increased in the GOS and CTP alone groups and the GOS/CTP 1:3 mixture treatment group compared with CON (p<0.05, FIG. 16).

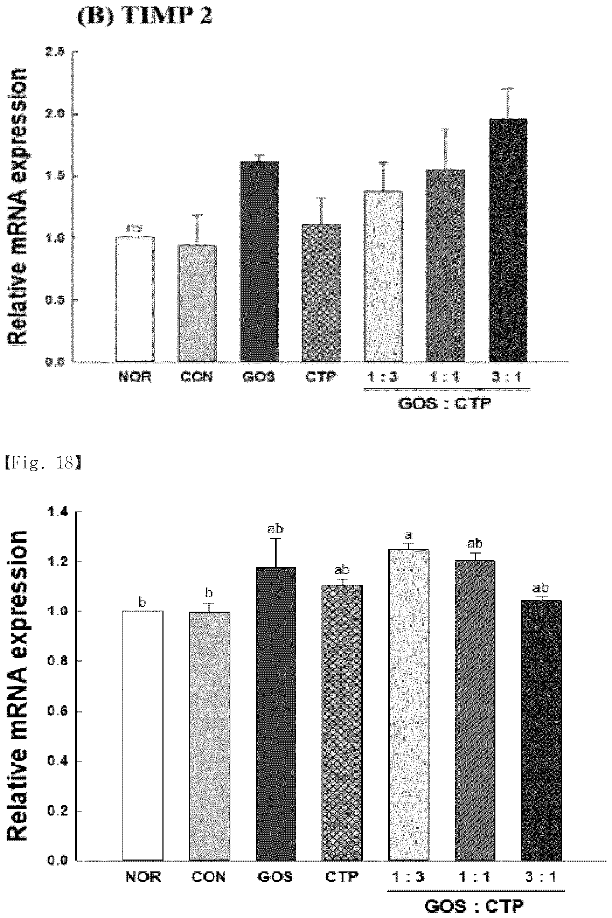

The expression level of TIMP 2 increased in the sample treatment groups compared with the CON group, but there was no significant difference between the groups. (FIG. 17).

It can be seen from the above results that the composition comprising GOS and the composition comprising GOS and CTP of the present disclosure promoted the expression of, especially, TIMP 1 to reduce the activity of MMPs and the degradation of extracellular matrix proteins thereby, and consequently suppressed wrinkle generation caused by photo-aging.

### Collagen type I (COL1a1)

As shown in FIG. 18, as a result of measuring the gene expression level of COL1a1, the CON group showed no difference in expression level of CoL1a1 compared with the NOR group, and the expression level of COL1A1 tended to increase in the group treated with the compositions of the present disclosure. Especially, the treatment with the composition comprising GOS/CTP at a ratio of 1:3 showed a significant increase in expression level of collagen type I by about 1.22 times compared with the CON group (p<0.05).

Among the extracellular matrix proteins in the skin connective tissue, collagen type I is the most abundant, and besides, elastin, fibronectin, integrin, fibrillin, proteoglycan, and the like are present. The newly synthesized procollagen is secreted into the extracellular space of skin cells through an enzymatic reaction, to thereby form microfibrils with a triple helix structure, and the microfibrils form collagen fibers to have connectivity and elasticity of the skin

The reduction of collagen fibers in the dermal layer due to photo-aging or intrinsic aging causes the dimpling of the skin tissue to result in wrinkles, the sagging of the skin, and the loss of elasticity.

It was considered from the above results that the treatment with the GOS composition and the GOS and CTP of the present disclosure increased the gene expression level of collagen type I, indicating that the compositions of the present disclosure were effective in wrinkle relief and elasticity enhancement.

### Hyaluronidase (HYAL)

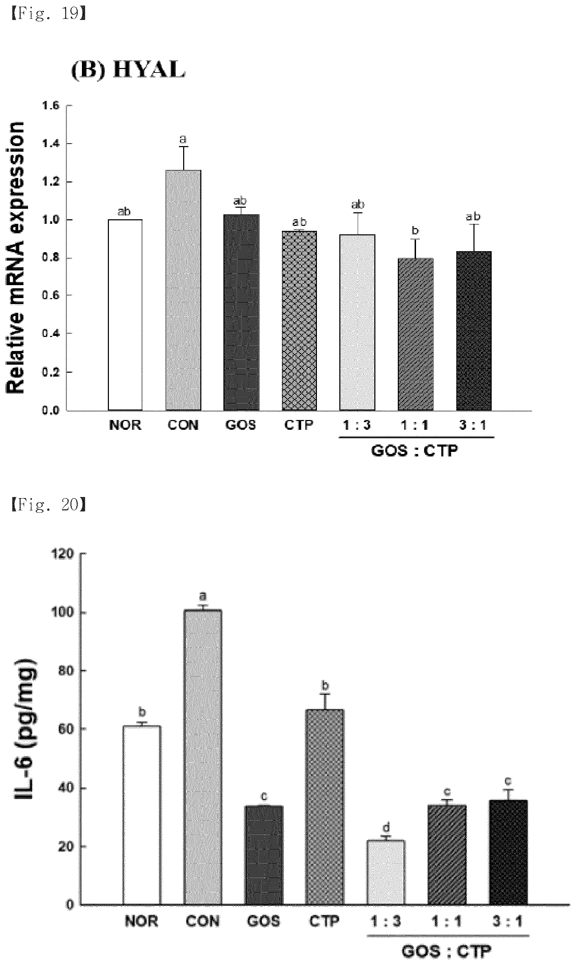

FIG. 19 shows the mRNA expression level of hyaluronidase of the skin tissue for each experimental group of the present disclosure.

As a result of measuring the gene expression level of the hyaluronidase HYAL gene, the expression level significantly increased by about 1.30 times in the CON group compared with the NOR group, and tended to decrease in all the composition administration groups (FIG. 19). Especially, the treatment with the composition comprising GOS/CTP mixed at a ratio of 1:3 showed a significant decrease in expression level of HYAL by about 0.61 times compared with the CON group (p<0.05).

### Example 8: Inflammation-related cytokine analysis using ELISA

In order to investigate the immune function improving or anti-inflammatory effect of the compositions of the present disclosure, the present inventors extracted the skin tissue to measure the contents of the inflammatory cytokines IL-6, IL-12, and TNF-α using the ELISA kit (BD Bioscience, San Jose, CA) for each experimental group. Protein levels were analyzed from tissue extracts through BCA method and the inflammation-related cytokines were expressed as contents per mg of protein.

As shown in FIG. 20, the interleukin-6 (IL-6) level significantly increased in the negative control group (CON) compared with the normal group (NOR), and decreased in the GOS/CTP 1:3 mixed treatment group compared with CON (p<0.05).

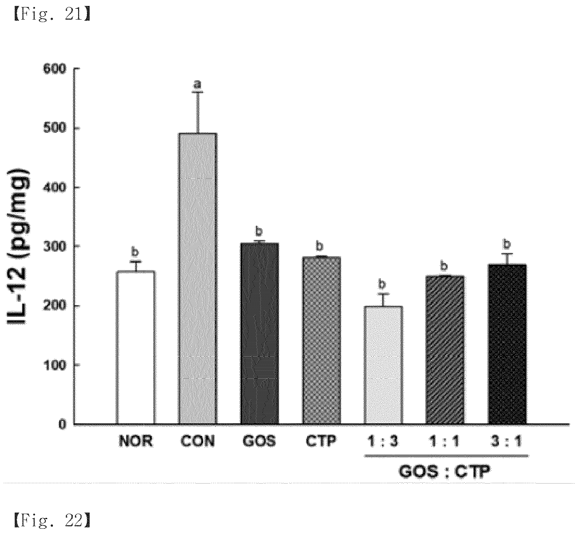

In addition, as shown in FIG. 21, as a result of measuring the interleukin-12 (IL-12) level, the IL-12 level significantly increased in the negative control group (CON) compared with the positive control group (NOR), and significantly decreased in all the composition administration groups compared with CON (p<0.05). The synergistic effect in the reduction of IL-12 secretion level was identified in all the mixture compositions, and especially, the GOS/CTP 1:3 mixed treatment group showed a largest decrease in IL-12 level.

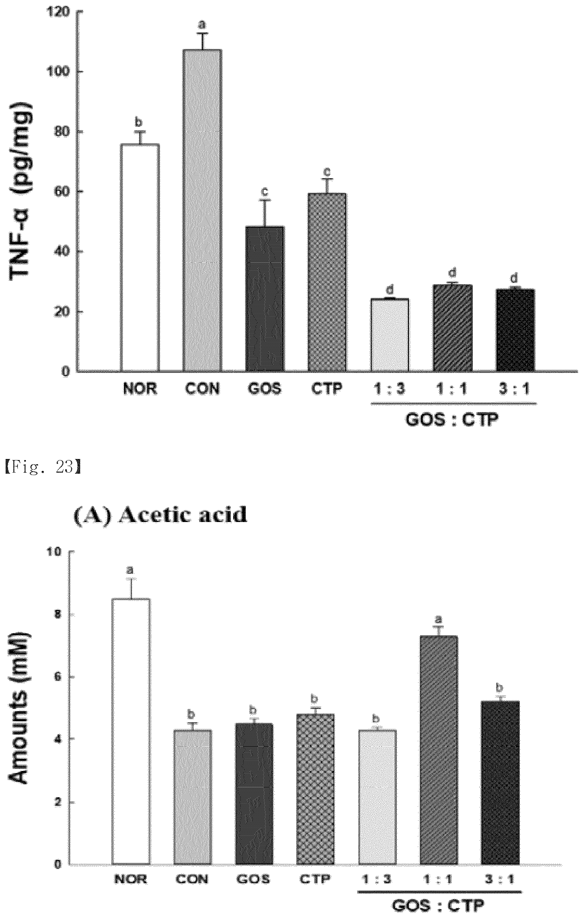

As shown in FIG. 22, as a result of measuring TNF-α, the TNF-α level significantly increased in the negative control group (CON) compared with the normal control group (NOR) (p<0.05). It was also identified from the above results that the TNF-α level significantly decreased in all the composition administration groups compared with the negative control group (CON), and a synergistic effect in the reduction of the TNF-α secretion level was identified in all the mixed compositions. Especially, a significant effect was identified in the GOS/CTP mixed administration groups (p<0.05).

It was identified from the above results that the reduction of the pro-inflammatory cytokines IL-6, IL-12, and TNF-α was identified in all the composition administration groups, and especially, a synergistic effect in the reduction of cytokines was identified when GOS/CTP were added in mixture. Therefore, the composition comprising GOS and the composition comprising GOS and CTP of the present disclosure reduce the inflammatory responses of the skin, and thus can be advantageously used as compositions for immune function improvement or anti-inflammation.

In an embodiment of the present disclosure, the composition for anti-inflammation shows a synergistic effect in all the ranges of GOS and CTP of 1:3 to 3:1.

### Example 9: Short-chain fatty acid (SCFA) analysis using GC

The caecum of the mouse was harvested and extracted with methanol, and then the extract was analyzed for SCFA by using gas chromatography (Agilent Technologies, CA, USA) equipped with GC column (DB-FFAP 123-3253, 50 m×0.32 mm×0.50 µM). The column temperature was set to be maintained at 100°C for 1 minute, raised to 180°C at a rate of 8 °C/min, then raised to 200°C at a rate of 20°C/min, and then stagnant at 200°C for 5 min. The SCFA contents in the caecum were analyzed through the standard materials of acetic acid, propionic acid, and butyric acid.

The results are shown in FIGS. 23 to 27.

As a result of analyzing the effect of GOS and CTP samples on the short-chain fatty acid generation by using gas chromatography, the content of acetic acid significantly decreased in the negative control group (CON) compared with the normal control group (NOR) (FIG. 23). However, the content of acetic acid significantly increased in the GOS/CTP 1:1 mixture treatment group compared with the CON group (p<0.05).

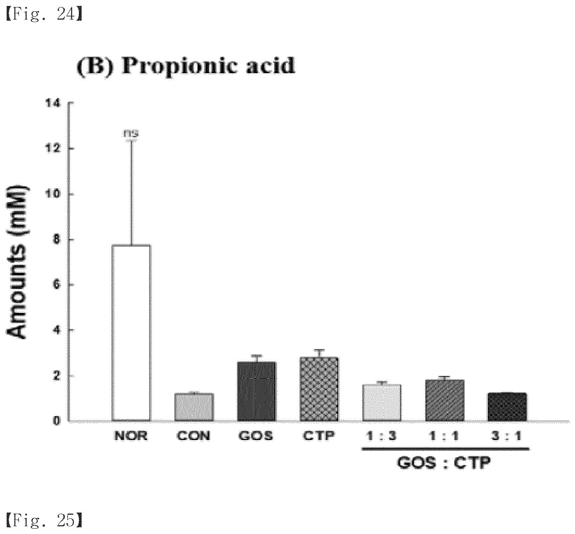

The content of propionic acid decreased in the negative control group (CON) compared with the normal control group (NOR), but there was no significant difference between the groups. (FIG. 24). The content of the propionic acid tended to increase in the GOS and CTP alone groups compared with the CON group, but there was no significant difference between the groups.

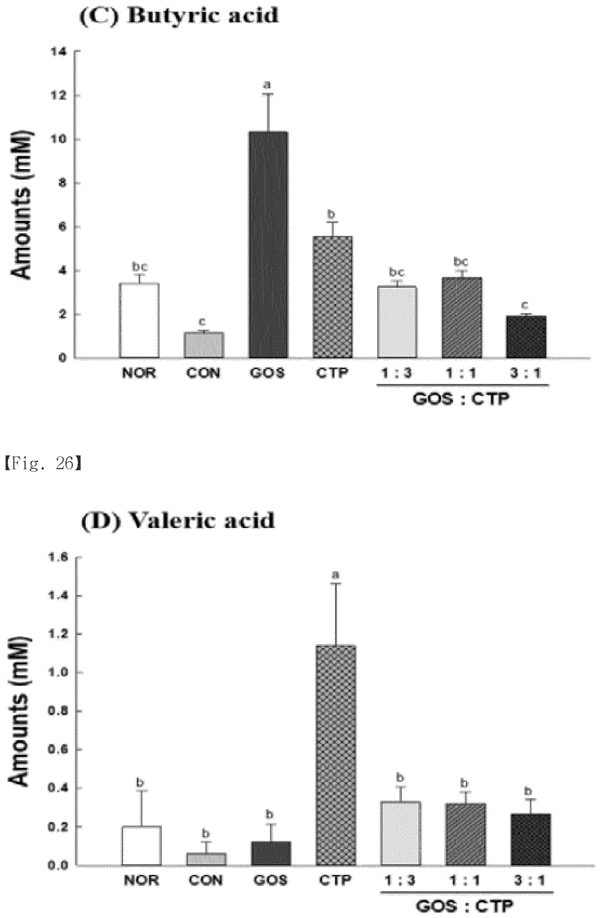

The content of butyric acid decreased in the negative control group (CON) compared with the normal control group (NOR) (FIG. 25). The content of butyric acid increased in the groups other than the GOS/CTP 3:1 mixture treatment group, and especially, significantly increased in the GOS and CTP alone treatment group.

The content of valeric acid tended to decrease in the negative control group (CON) compared with the normal control group (NOR) (FIG. 26), and among the sample groups, the CTP alone treatment group showed a significant increase in valeric acid content compared with CON (p<0.05).

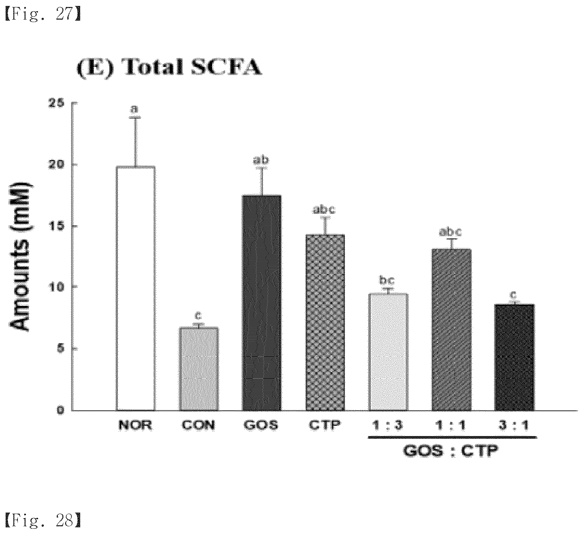

As a result of analyzing total short-chain fatty acid content, the negative control group (CON) showed a significant low content compared with the normal control group (NOR), and the GOS alone treatment group showed a significant high content compared with the CON group (p<0.05), and the CTP alone and GOS/CTP 1:1 mixture treatment group showed a tendency of increase (FIG. 27).

It is known that the fermentation of dietary fibers in the intestine by intestinal microbes produces short-chain fatty acids, mainly acetic acid, propionic acid, and butyric acid, and these short-chain fatty acids are promptly absorbed into the intestinal mucosa and used as a main energy source for colonic mucosal cells (Thompson DB, 2000).

As a result of the present study, short-chain fatty acids were significantly reduced due to photo-aging, and thus the correlation between the skin and intestinal microbes was identified, and against such reductions, the shot-chain fatty acids were increased by the intake of GOS and CTP, and thus the correlations thereof with intestinal environment improvement and skin protecting effects were identified. Through future research, the change in the intestinal microflora is to be analyzed to evaluate the correlation between the skin and intestinal microbes and the effect of the intake of GOS and CTP on the intestinal environment change.

### Example 10: Intestinal microflora change analysis

The caecum of the mouse was harvested, and subjected to extraction using the i-genome clinic DNA extraction kit. The 16S rRNA library was constructed through the protocol of Illumina 16S V3-V4 Amplicon (Illumina, San Diego, CA, USA). The 16S rRNA V3-V4 region was amplified by the first polymerase chain reaction (PCR-1) using the DNA extract, and the PCR product was purified using Agencourt AMPure XP Beads (Beckman Coulter, Inc., Pasadena, CA, USA), and the washed amplicons were quantified. The PCR product was used as a template for the second PCR (PCR-II) with Illumina dual index adapter sequence, and the final amplicon library was sequenced using the v3, 600 cycle kit and 301 base paired-end chemistry. Sequence data analysis was performed through QIIME 2 software, and then quality filtering was performed. The homopolymers were removed from the QIIME pipeline, and a representative sequence set was selected using UCLUST at a similarity level of 97.0%, and clustering was performed. The operational taxonomic units (OTUs) of the sequence were analyzed, and the alpha diversity analysis included the Richness, Shannon Index, and Abundance-based Coverage Estimator (ACE) indexes. For the beta diversity within and between groups, the UniFrac measurement distance was analyzed, and these results were expressed by the principal coordinate analysis (PCoA). As for the variables in the group having the same composition of the intestinal microflora composition (OTU), the correlations between means were identified using Pearson Correlation. The Kruskal-Wallis test was performed to analyze the diversity of the intestinal microflora and the difference in relative abundance, and the difference in beta diversity was analyzed by the ANOSIM and permutational multivariate analysis of variance (PERMANOVA) tests.

**TABLE 4**

| Microbiome analysis (Alpha diversity summary) | | | | |
|---|---|---|---|---|
| Sample name | OTUs | ACE | CHAO | Jackknife |
| NOR 1 | 595 | 683.32 | 667.23 | 705.13 |
| NOR 2 | 658 | 706.74 | 699.64 | 737 |
| NOR 3 | 712 | 773.93 | 759.5 | 807 |
| CON 1 | 592 | 653.31 | 642.7 | 682 |
| CON 2 | 742 | 827.06 | 804.16 | 854 |
| CON 3 | 727 | 806.72 | 802.37 | 838.69 |
| GOS1 | 624 | 695.04 | 684.6 | 726 |
| GOS2 | 613 | 681.21 | 670.99 | 708 |
| GOS3 | 677 | 747.72 | 745.24 | 778.55 |
| CTP1 | 611 | 688.92 | 677.25 | 717 |
| CTP2 | 665 | 723.52 | 722.55 | 752 |
| CTP3 | 733 | 821.20 | 816.38 | 852.19 |
| GOS/CTP 1:3 (1) | 606 | 679.37 | 678.14 | 710.12 |
| GOS/CTP 1:3 (2) | 723 | 776.36 | 759.4 | 804 |
| GOS/CTP 1:3 (3) | 570 | 655.82 | 693.68 | 805.62 |
| GOS/CTP 1:1 (1) | 608 | 691.31 | 680.27 | 714.72 |
| GOS/CTP 1:1 (2) | 663 | 732.80 | 729.23 | 757.65 |
| GOS/CTP 1:1 (3) | 688 | 780.67 | 790.06 | 833.56 |
| GOS/CTP 3:1 (1) | 610 | 702.50 | 704.25 | 730.08 |
| GOS/CTP 3:1 (2) | 640 | 700.14 | 697.55 | 727 |
| GOS/CTP 3:1 (3) | 675 | 1741.76 | 744.72 | 777.07 |

| | | | | |
|---|---|---|---|---|
| NOR: normal group; CON: UVB-control group. Data are expressed as means ± standard error (n=6) and the different letters indicate significant differences at p<0.05 by Tukey's test. | | | | |

**TABLE 5**

| Sample name | NPShannon | Shannon | Simpson | Phylogenetic Diversity | Good's coverage of library(%) |
|---|---|---|---|---|---|
| NOR 1 | 4.03 | 4.01 | 0.06 | 803 | 99.71 |
| NOR 2 | 4.15 | 4.15 | 0.05 | 927 | 99.89 |
| NOR 3 | 4.04 | 4.02 | 0.09 | 920 | 99.83 |
| CON 1 | 4.51 | 4.49 | 0.03 | 794 | 99.75 |
| CON 2 | 4.32 | 4.31 | 0.05 | 930 | 99.81 |
| CON 3 | 4.26 | 4.25 | 0.06 | 928 | 99.82 |
| GOS1 | 4.39 | 4.37 | 0.04 | 827 | 99.71 |
| GOS2 | 3.66 | 3.65 | 0.09 | 872 | 99.86 |
| GOS3 | 3.91 | 3.9 | 0.08 | 911 | 99.86 |
| CTP1 | 4.26 | 4.25 | 0.06 | 856 | 99.71 |
| CTP2 | 4.2 | 4.19 | 0.05 | 931 | 99.89 |
| CTP3 | 4.42 | 4.41 | 0.04 | 968 | 99.81 |
| GOS/CTP 1:3 (1) | 4.55 | 4.53 | 0.03 | 821 | 99.72 |
| GOS/CTP 1:3 (2) | 4.71 | 4.7 | 0.02 | 989 | 99.89 |
| GOS/CTP 1:3 (3) | 3.36 | 3.35 | 0.1 | 788 | 99.78 |
| GOS/CTP 1:1 (1) | 4.64 | 4.62 | 0.03 | 825 | 99.71 |
| GOS/CTP 1:1 (2) | 4.49 | 4.49 | 0.04 | 959 | 99.88 |
| GOS/CTP 1:1 (3) | 4.65 | 4.64 | 0.03 | 937 | 99.81 |
| GOS/CTP 3:1 (1) | 4.08 | 4.06 | 0.07 | 833 | 99.71 |
| GOS/CTP 3:1 (2) | 4.07 | 4.06 | 0.06 | 885 | 99.87 |
| GOS/CTP 3:1 (3) | 3.69 | 3.68 | 0.1 | 905 | 99.83 |

| | | | | | |
|---|---|---|---|---|---|
| NOR: normal group; CON: UVB-control group. Data are expressed as means ± standard error (n=6) and the different letters indicate significant differences at p<0.05 by Tukey's test. | | | | | |

Tables 4 and 5 show the overall summary of alpha diversity in the population analysis for 21 samples. The means of respective assay values were found: OTUs 653.90, ACE 727.12, CHAO 722.38, Jackknife 762.73, NPShannon 4.21, Shannon 4.20, Simpson 0.06, phylogenetic diversity 886.14, and Good's coverage of library (%) 99.80.

### Bacterial population comparison at genus level

As a result of bacterial population analysis at the genus level, 470 taxa were found and classified as 50 representative taxa. The compositions of the bacterial population in the analyte samples were listed in the order of appearance as follows.

Akkermansia, PAC000661_g, Eubacterium_g23, PAC000664_g, PAC000198_g, Sporobacter, Bacteroides, Lactobacillus, Pseudoflavonifractor, Eubacterium_g17, PAC002367_g, Parabacteroides, Oscillibacter, Ruminococcus.

Among the top 20 taxa at the genus level, the genus Akkermansia corresponding to the phylum Verrucomicrobia was detected at 10% or more in 10 samples (N1, CTP1, CTP2, CTP3, GOS/CTP 1:3 (1), (3), GOS/CTP 1:1 (1), (2), GOS/CTP 3:1 (1), (3)) and detected in 22.77% in GOS/CTP 1:3 (3) sample. However, the genus Akkermansia was detected at 0.2% or less in all the CON samples, showing the lowest value among the samples.

The genus Bacteroides was detected at 10% or more in seven samples (N2, GOS1, 2, CTP3, GOS/CTP 1:3 (3), GOS/CTP 3:1 (1), (3)). Like the genus Akkermansia, the genus Bacteroides was detected at 28.73% in the GOS/CTP 1:3 (3) sample, showing the highest value, and detected in 3.11% in CON1, showing the lowest value.

### Comparison of relationships between samples

### PCoA assay

To analyze the relationship of GOS and CTP with the dermal and intestinal health functional microbiomes, principal coordinate analysis (PCoA: principal component) was performed using Unifrac distance metrics for the population results at the genus level. The population of 21 samples were scattered and did not form a specific group.

### UPGMA analysis

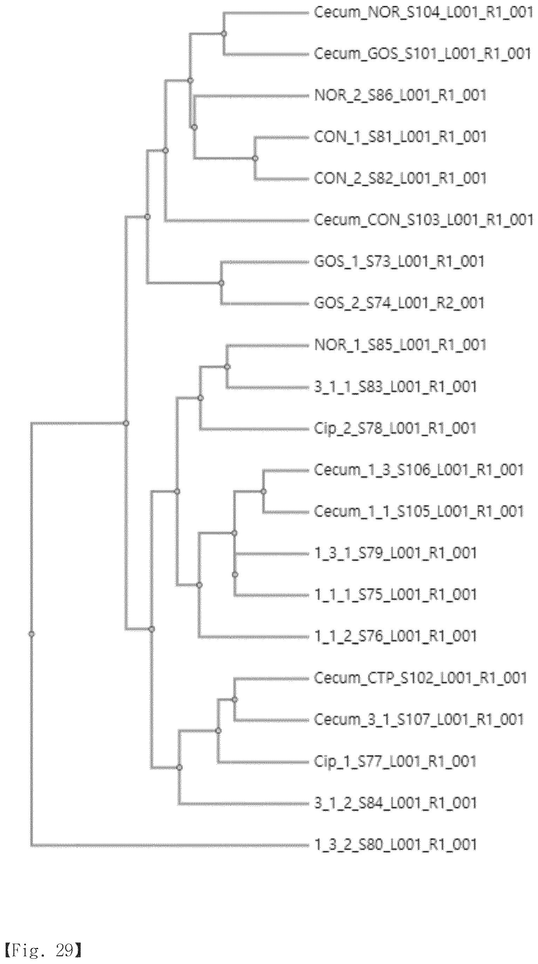

As a result of UPGMA clustering analysis, samples with similar population compositions and samples with different population compositions were identified in each group (FIG. 28). Relatively, the population composition was similar among the NOR, CON, and GOS groups, especially, between the NOR and GOS groups, and similar between the CTP group and the GOS/CTP mixed group.

### Changes in intestinal microflora

### Changes in Bacteroidetes and Firmicutes

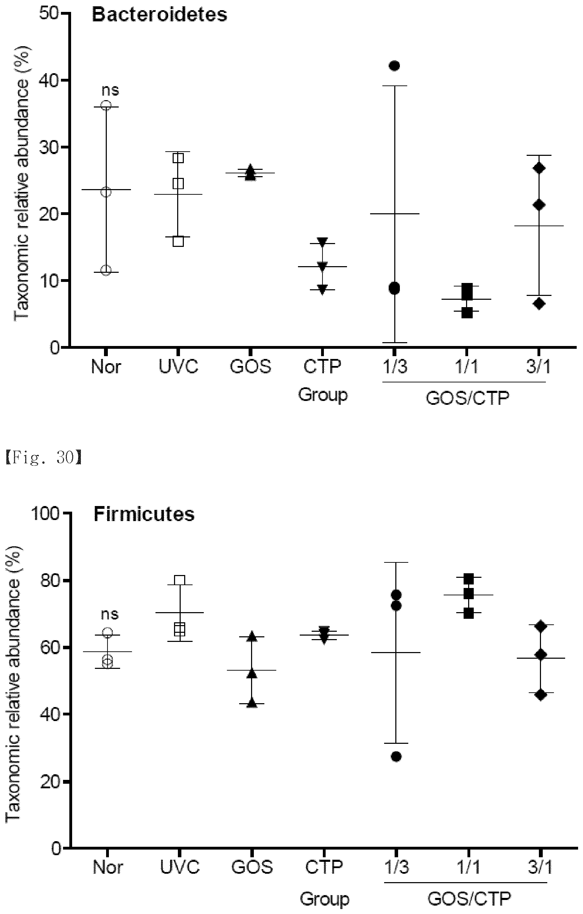

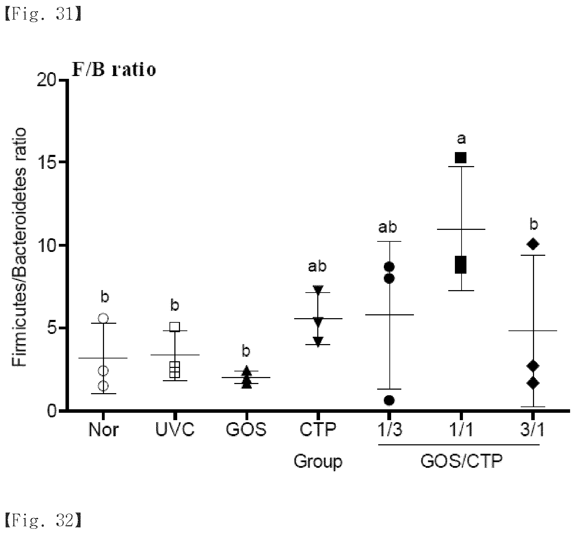

The changes in Bacteroidetes and Firmicutes in the phylum by the oral administration of the GOS/CTP mixture were measured, respectively (FIGS. 29 to 31). The human intestinal microflora includes 50 or more different phyla and more than 1000 or more different species of bacteria. Of these, the five dominant phyla are: Firmicutes, to which Lactobacillus belongs, Bacteroidetes, Actinobacteria , to which Bifidobacterium belongs, Proteobacteria, and Verrucomicrobia. Of these, the two phyla, Bacteroidetes and Firmicutes, are dominant and account for more than 80% of the normal intestinal microbes. Despite such diversity of microbial species, only 18 key species are present in all the individuals. In addition, the ratio of the two dominant phyla varies from individual to individual, and the Firmicutes:Bacteroidetes ratio was observed to be changed in diseased patients, and thus is considered to be related to disease development.

The Firmicutes/Bacteroidetes ratio is used as an indicator of decreased and increased diversity of the intestinal microflora. The patients with irritable bowel syndrome show a decrease in diversity of the intestinal microflora and decreased Bifidobacterium and Lactobacillus, and a tendency of the increase in Fermicutes:Bacteroidetes ratio (Kim et al., 2013; Hong and Rhee, 2014; Tojo et al., 2014; and Dupont, 2014).

When GOS and CTP alone or the GOS/CTP mixtures were orally administered to the photo-aging models, Firmicutes decreased and the Firmicutes/Bacteroidetes ratio also tended to decrease in the administration of GOS. The F/B ratio tended to increase in the administration of CTP, with no significant difference compared with CON, and the F/B ratio decreased with the increase in GOS concentration in the GOS/CTP mixtures. It is considered from such results that the administration of GOS is involved in the intestinal environment improvement.

### Changes in lactic acid bacteria

The changes in lactic acid bacteria, among the intestinal microbes, according to GOS/CTP intake were measured, and Lactobacillus and Bifidobacterium were identified as main strains among all the lactic acid bacteria. Bifidobacterium showed a tendency to increase in the administration of GOS. The administration of the GOS/CTP 1:3 mixture resulted in a significant increase in Bifidobacterium compared to the CON group (p<0.05). As for the change in the Lactobacillus species belonging to lactic acid bacteria, there was no significant difference in each group. The change in Lactococcus species tended to increase by the administration of GOS, and tended to rather decrease in the GOS/CTP mixtures excluding the 1:1 administration group, compared with the CON group, but there was no significant difference.

### Changes in human intestinal microbes

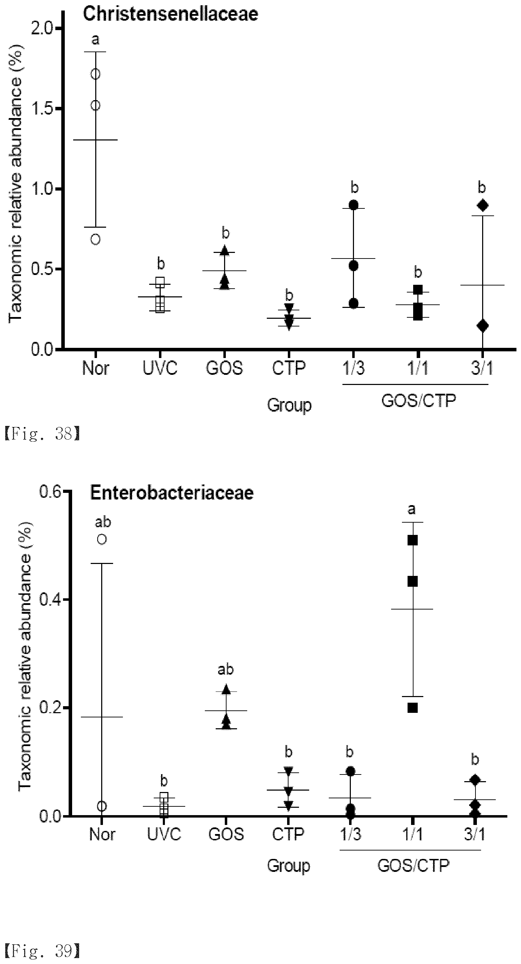

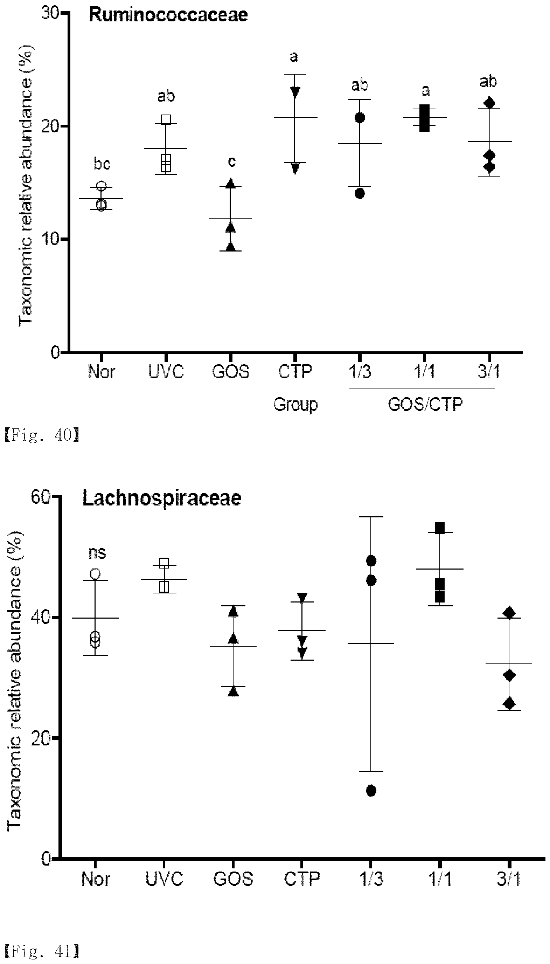

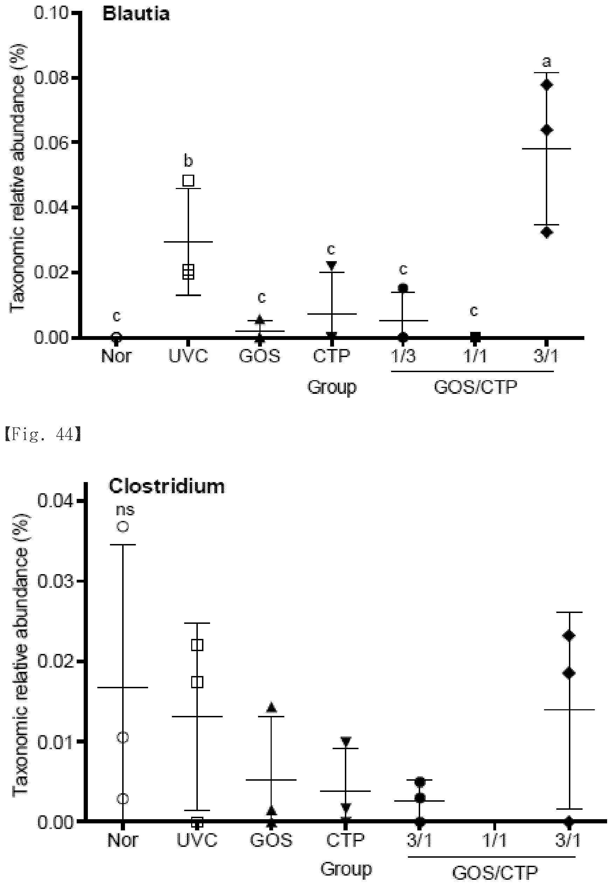

The results of measuring changes in representative microbes observed in the human intestine are shown in FIGS. 38 to 46.

The changes in microbes belonging to the phyla and families of the human intestine were measured. Proteobacteria, which are microbes belonging to the phylum, are observed at low levels in the healthy intestine and tend to increase in the administration of the GOS/CTP mixtures, and especially, showed a significant increase in the 1:3 group compared with the CON group (p<0.05). The intestinal bacteria Christensenellaceae were mainly found in slender twins. Although there was a report that the mice did not gain weight even after eating a high-fat, nutrient-rich diet when the intestinal bacteria Christensenellaceae were injected into the mice, but Christensenellaceae rather tended to decrease in the sample treatment groups compared with the normal control group (NOR). The diversity of the intestinal microflora is known to decrease in old age (O'Toole and Claesson, 2010). That is, it is known that Bifidobacteriaceae decrease and Enterobacteriaceae increase (Woodmansey, 2007; and Claesson et al., 2011).

The GOS/CTP mixture administration groups of the present disclosure, especially, 1:3 and 3:1 groups showed a tendency of decrease in Enterobacteriaceae.

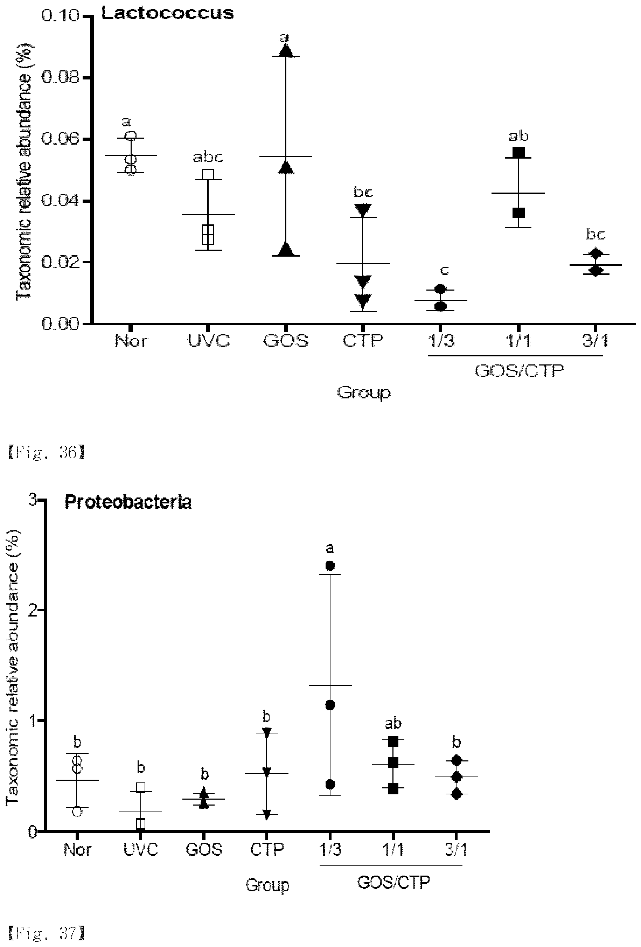

FIGS. 36 to 40 show the distributions of the phyla and families of intestinal microorganisms in the intestinal microbiomes for each experimental group of the present disclosure.

Overall, Proteobacteria are low, but Bacteriodes, Prevotella, and Ruminococcus are abundant and thus considered to be healthy gut microorganisms (Kim et al., 2013; and Tojo et al., 2014). The genera Bacteroides, Prevotella, and Xylanibacter belong to the phylum Bacteroidetes, and can degrade various types of glycan complexes. Ruminococcus generally tended to increase by the administration of GOS and CTP, and especially, significantly increased in the GOS/CTP 1:1 mixture administration group compared with the NOR group (p<0.05).

Individuals are classified according to the dominant species of bacteria distributed in the large intestine (enterotype), and may be classified into three enterotypes: enterotype 1 for dominant Bacteroides, enterotype 2 for dominant Prevotella, and enterotype 3 for dominant Ruminococcus. The distribution patterns of the classified enterotypes are known to vary according to the dietary intake, genetic differences, and residential area, but the meanings thereof are still controversial (Bennet et al., 2015; and Eupont, 2014).

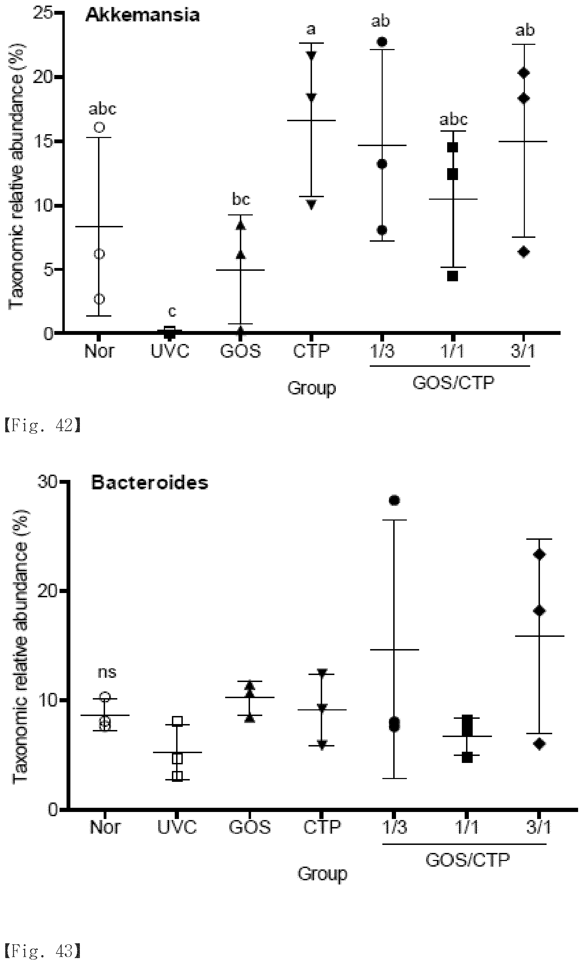

The results of measuring the changes in representative microorganisms (genus) observed in the human intestine are shown in FIGS. 41 to 44. Akkermansia decreased in the UV control group (CON) compared with the NOR group, and increased in the sample treatment groups, showing a similar level to that of the NOR group. Blautia significantly increased in the CON group, and tended to decrease in the treatment with most of the samples. Bacteroides and Clostridium showed no significant differences between the samples. The change in the intestinal flora was identified in the intake of GOS/CTP mixtures, and the administration of GOS showed a slight improvement effect in the ratio of Firmicutes/Bacteroidetes. In addition, the intestinal lactic acid bacteria were identified to increase in the GOS/CTP mixture administration groups showing a skin improvement effect in the photo-aging models.

## Claims

1. Use of a food composition comprising galacto-oligosaccharides (GOS) and collagen tripeptide (CTP) for relieving skin wrinkles, wherein the galacto-oligosaccharides and the collagen tripeptide (CTP) are present at a weight ratio of 1:3.

## Patentansprüche

1. Verwendung einer Lebensmittelzusammensetzung umfassend Galacto-Oligosaccharide (GOS) und Kollagentripeptid (CTP) zur Linderung von Hautfalten, wobei die Galacto-Oligosaccharide und das Kollagentripeptid (CTP) in einem Gewichtsverhältnis von 1:3 vorliegen.

## Revendications

1. Utilisation d'une composition alimentaire comprenant des galacto-oligosaccharides (GOS) et du tripeptide de collagène pour atténuer les rides de la peau, dans laquelle les galacto-oligosaccharides (GOS) et le tripeptide de collagène sont présents dans un ratio massique de 1 : 3.
